(19) Europäisches Patentamt
European Patent Office
Office européen des brevets

(11) EP 0 610 487 B1

(12) EUROPÄISCHE PATENTSCHRIFT

(45) Veröffentlichungstag und Bekanntmachung des Hinweises auf die Patenterteilung:
**10.11.1999 Patentblatt 1999/45**

(21) Anmeldenummer: **93919208.4**

(22) Anmeldetag: **28.08.1993**

(51) Int Cl.[6]: **C07K 5/06**, C07K 5/02

(86) Internationale Anmeldenummer:
**PCT/EP93/02329**

(87) Internationale Veröffentlichungsnummer:
**WO 94/05693 (17.03.1994 Gazette 1994/07)**

# (54) NEUE AMINOSÄUREDERIVATE, VERFAHREN ZU IHRER HERSTELLUNG UND DIESE VERBINDUNGEN ENTHALTENDE PHARMAZEUTISCHE ZUSAMMENSETZUNGEN

NEW AMINOACID DERIVATES, PROCESS FOR PRODUCING THE SAME AND PHARMACEUTICAL COMPOSITIONS CONTAINING THESE COMPOUNDS

NOUVEAUX DERIVES D'ACIDES AMINES, LEUR PROCEDE DE PRODUCTION ET COMPOSITIONS PHARMACEUTIQUES CONTENANT CES COMPOSES

(84) Benannte Vertragsstaaten:
**AT BE CH DE DK ES FR GB GR IE IT LI LU MC NL PT SE**

(30) Priorität: **03.09.1992 DE 4229447**
**22.12.1992 DE 4243496**
**08.05.1993 DE 4315437**

(43) Veröffentlichungstag der Anmeldung:
**17.08.1994 Patentblatt 1994/33**

(60) Teilanmeldung: **99100929.1**

(73) Patentinhaber:
- **Boehringer Ingelheim Pharma KG**
**55216 Ingelheim am Rhein (DE)**
Benannte Vertragsstaaten:
**BE CH DE DK ES FR GR IT LI LU MC NL PT SE AT**
- **Boehringer Ingelheim International GmbH**
**55216 Ingelheim (DE)**
Benannte Vertragsstaaten:
**GB IE**

(72) Erfinder:
- **ESSER, Franz**
**D-55218 Ingelheim am Rhein (DE)**
- **SCHNORRENBERG, Gerd**
**D-55435 Gau-Algesheim (DE)**
- **DOLLINGER, Horst**
**D-55218 Ingelheim am Rhein (DE)**
- **JUNG, Birgit**
**D-55411 Bingen am Rhein (DE)**
- **BÜRGER, Erich**
**D-55411 Bingen am Rhein (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 394 989**
**EP-A- 0 443 132**
**EP-A- 0 482 539**
**EP-A- 0 515 681**
**EP-A- 0 517 589**
**WO-A-92/22569**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 610 487 B1

**Beschreibung**

Die Erfindung betrifft neue Aminosäurederivate der allgemeinen Formel I,

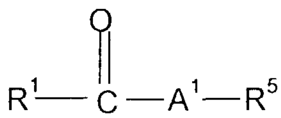

(I)

und deren pharmazeutisch annehmbare Salze, Verfahren zu ihrer Herstellung und diese Verbindungen enthaltende pharmazeutische Zusammensetzungen. Die Verbindungen sind wertvolle Neurokinin (Tachykinin)-Antagonisten.

In den europäischen Patentanmeldungen EP 394 989 und EP 443 132 werden Peptide mit Neurokinin antagonistischer Wirkung beschrieben. Die erfindungsgemäßen Verbindungen unterscheiden sich von diesen Peptiden wesentlich in dem Glied $R^5$.

Die in dieser Beschreibung und den Ansprüchen für die Aminosäuren verwendeten Abkürzungen entsprechen dem üblichen Dreibuchstabencode wie er z.B. in Europ. J. Biochem., 138, 9 (1984) beschrieben ist. Die übrigen Abkürzungen werden nachfolgend erklärt:

| | |
|---|---|
| Boc = | t-Butoxycarbonyl |
| Bzl = | Benzyl |
| CDI = | Carbonyldiimidazol |
| Cha = | 3-Cyclohexylalanin |
| DCCI = | Dicyclohexylcarbodiimid |
| DCH = | Dicyclohexylharnstoff |
| HOBt = | 1-Hydroxybenztriazol |
| Hpa = | Homophenylalanin |
| Hyp = | (2S,4R)-Hydroxyprolin |
| Pal = | 3-(1-Pyrrolyl)alanin |
| THF = | Tetrahydrofuran |
| TFA = | Trifluoressigsäure |
| Z = | Benzyloxycarbonyl |
| Me = | Methyl |
| Ac = | Acetyl |
| Et = | Ethyl |
| DMF = | Dimethylformamid |
| DPPA = | Diphenylphosphorylazid |
| PPA = | Polyphosphorsäure |
| RT = | Raumtemperatur |

Der Ausdruck Aminosäure umfaßt (falls im folgenden Text nicht ausdrücklich etwas anderes angegeben ist) natürliche und unnatürliche Aminosäuren, sowohl der D-als auch der L-Form, insbesondere α-Aminosäuren, sowie deren Isomere.

Wenn eine Aminosäure ohne Präfix angegeben ist (z.B. Orn) steht diese Angabe für die L-Form der Aminosäure. Die D-Form wird ausdrücklich angegeben.

Die Erfindung betrifft neue Aminosäurederivate der allgemeinen Formel I

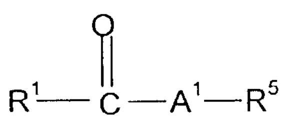

(I)

und deren pharmazeutisch annehmbare Salze, worin

$R^1$ Vinyl, Aryl, Heteroaryl, Aralkyl, Heteroaralkyl, Arylvinyl, Heteroarylvinyl, Aryloxyalkyl, Arylalkyloxy, $(C_3$-$C_8)$cyclo-

alkyl, ($C_3$-$C_8$) Cycloalkylalkyl, unsubstituiertes oder durch 1-3 Methylgruppen substituiertes Bicycloheptyl oder Bicycloheptylalkyl, Adamantyl, Adamantylalkyl, Dekalin, Dekalinalkyl, Tetralin, Tetralinalkyl, Diphenylalkyl, Di(arylalkyl)- aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl, Hydroxy, Nitro, Alkylcarbonyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin ($\Delta$Pro) wie beispielsweise 3,4-Didehydroprolin ($\Delta$(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin (Pro($NH_2$)) wie beispielsweise 3-Aminoprolin (Pro(3$NH_2$)) und 4-Aminoprolin (Pro(4$NH_2$)), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), Tpr ($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$R^5$ für ein Amin der Formel

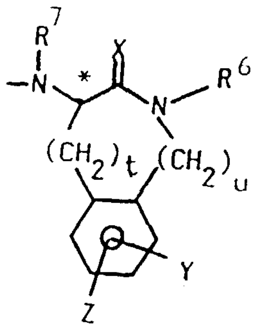

II

oder

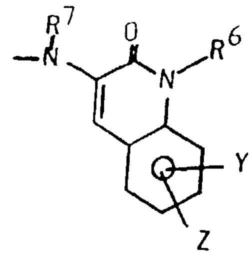

III

steht, worin

$R^6$ für Aralkyl, Diarylalkyl (in diesen Gruppen ist Aryl Phenyl oder Naphthyl und Alkyl ($C_1$-$C_5$) Alkyl)), Heteroaryl-($C_1$-$C_5$)-alkyl (worin Heteroaryl 2-,3- oder 4-Pyridyl oder 2- oder 3-Thienyl ist), Phenylamino-($C_1$-$C_5$)-alkyl, Nephthylamino-($C_1$-$C_5$) -alkyl oder N-Phenylalkylpiperidinyl (worin die angeführten Phenylgruppen unsubstituiert sind oder 1, 2 oder 3 Substituenten enthalten, die unabhängig voneinander ($C_1$-$C_5$)-Alkyl, vorzugsweise Methyl, ($C_1$-$C_5$)-Alkoxy, vorzugsweise Methoxy, Dimethylamin, Halogen, Trifluormethyl, -CN oder $OCF_3$ sind) steht;

$R_7$ für Wasserstoff oder ($C_1$-$C_5$)-Alkyl steht;

X für 0 oder $H_2$ steht;

Y und Z unabhängig voneinander für Wasserstoff ($C_1$-$C_5$)-Alkyl, ($C_1$-$C_5$)-Alkyloxy, Benzyloxy (worin die Phenylgruppe unsubstituiert ist oder 1, 2 oder 3 Substituenten enthält, die unabhängig voneinander ($C_1$-$C_5$)-Alkyl, vorzugsweise Methyl, ($C_1$-$C_5$)- Alkoxy, vorzugsweise Methoxy, Dimethylamin, Halogen, Trifluormethyl, -CN oder $OCF_3$ sind), $OCF_3$, Halogen, $CF_3$, CN, $CH_2NH_2$, $CONH_2$, N-($C_1$-$C_5$-Alkyl)$_2$, NH-($C_1$-$C_4$)-alkylcarbonyl, N-($C_1$-$C_5$)-Alkyl-N-($C_1$-$C_4$)-alkylcarbonyl, $NH_2$ oder NH-($C_1$-$C_5$)-Alkyl stehen oder wenn Y und Z vicinal zueinander stehen, diese

zusammen $-OCH_2O-$, $OCH_2CH_2O-$ oder $(CH)_4$ bedeuten;

t und u eine der folgenden Bedeutungen haben

(a) t und u null
(b) t eins und u null
(c) t und u je eins
(d) t zwei und u null;

und wenn t eins und u null ist, $R^5$ auch für ein Amin der Formel IV

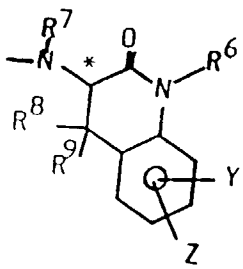

IV

steht, worin

$R^6$, $R^7$, Y und Z die oben genannte Bedeutung haben und

$R^8$ für Wasserstoff steht und $R^9$ für Hydroxy, $(C_1$-$C_5)$-Alkoxy, Phenyl-$(C_1$-$C_5)$-alkyloxy, Naphthyl-$(C_1$-$C_5)$-alkyloxy oder $(C_1$-$C_4)$-Alkylcarbonyl, oder worin

$R^8$ und $R^9$ zusammen für Sauerstoff oder $-OCH_2CH_2O-$ stehen;

und die Chiralität an C* R oder S bedeuten kann.

Verbindungen der allgemeinen Formel I können Säuregruppen besitzen , hauptsächlich Carboxylgruppen, oder phenolische Hydroxygruppen, und/oder basische Gruppen wie z.B. Guanidino- oder Aminofunktionen. Verbindungen der allgemeinen Formel I können deshalb entweder als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die Chiralitätszentren in den neuen Aminosäurederivate können jeweils R-, S- oder R,S-Konfiguration besitzen.

Von den erfindungsgemäßen Verbindungen der Formel I sind solche bevorzugt, worin

$R^1$ Vinyl, Aryl, Heteroaryl, Aralkyl, Heteroaralkyl, Arylvinyl, Heteroarylvinyl, Aryloxyalkyl, Arylalkyloxy, Di(arylalkyl) aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D- oder L-Threonin (Thr), D- oder L-alloThreonin, D- oder L-Cystein (Cys), D-oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin ($Pro(NH_2)$) wie beispielsweise 3-Aminoprolin ($Pro(3NH_2)$) und 4-Aminoprolin ($Pro(4NH_2)$), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin

(Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), TPr(O) , Met(O), Tpr($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können.

Von den erfindungsgemäßen Verbindungen der Formel I sind insbesondere solche bevorzugt worin

$R^1$ Aryl, Heteroaryl, Aralkyl, Heteroaralkyl, Aryloxyalkyl, Arylalkyloxy, Di(arylalkyl)aminoalkyl (worin Aryl für Phenyl oder 1-oder 2-fach substituiertes Phenyl steht; die Substituenten der Phenylgruppe unabhängig voneinander Halogen oder Alkoxy sind; Heteroaryl für Indolyl, in Stellung 1 durch Alkyl oder Benzyl substituiertes Indolyl oder Pyridyl steht; und die Alkyl- bzw. Alkoxygruppe 1 bis 3 Kohlenstoffatome enthält) bedeutet; insbesondere

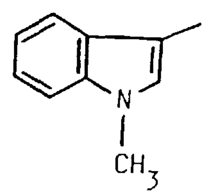

und/oder

$A^1$ eine Aminosäure ist, die eine oder 2 polare funktionelle Gruppe(n) in der Seitenkette trägt, wie OH, COOH, $NH_2$, Guanidin, $CONH_2$, SH; insbesondere worin
die funktionelle Gruppe in der Seitenkette von $A^1$ OH ist und/oder worin $A^1$ Pro, 4-Hydroxyprolin, 3-Hydroxyprolin, Ser, Thr, Trp(For) oder Tyr ist; vorzugsweise 4-Hydroxyprolin mit 2-S-Konfiguration ist, insbesondere

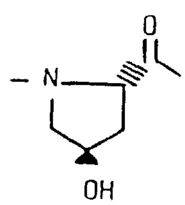 .

Von den erfindungsgemäßen Verbindungen sind solche bevorzugt, worin $R^5$ eine Gruppe der allgemeinen Formel II

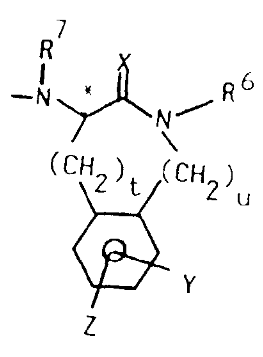

II

ist, insbesondere solche, worin t eins und u null oder t zwei und u null oder t und u je eins ist, und $R^6$, $R^7$, X, Y und Z

wie oben definiert sind.

Hervorzuheben sind solche Verbindungen, worin $R^6$ Benzyl oder Methoxybenzyl ist und/oder worin $R^7$ Wasserstoff ist und/oder worin X Oxo ist und/oder worin Y und Z unabhängig voneinander Methoxy, Wasserstoff, $CF_3$ oder tert. Butyl sind oder zusammen $-(CH)_4-$bedeuten.

Die angegebenen Aminosäuren liegen vorzugsweise in S-Konfiguration vor.

Untersuchungsergebnisse für erfindungsgemäße Verbindungen:

Die Rezeptoraffinität zum $NK_1$-Rezeptor (Substanz P-Rezeptor) wurde an humanen Lymphoblastoma-Zellen (IM-9) mit klonierten $NK_1$-Rezeptoren bestimmt, wobei die Verdrängung von $^{125}J$-markierter Substanz P gemessen wird. Die so erhaltenen $IC_{50}$-Werte betragen:

| | |
|---|---|
| Verb. E | 6 nM |
| Verb. F | 15 nM |
| Verb. G | 1,7 nM |

Verbindung E

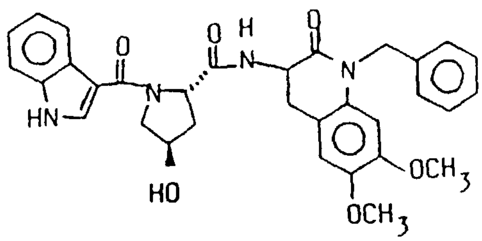

Verbindung F

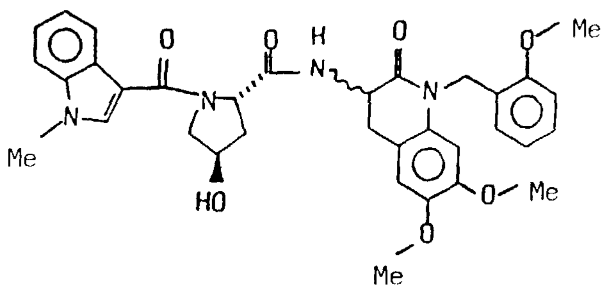

(2S, 4R)S-Form

Verbindung G

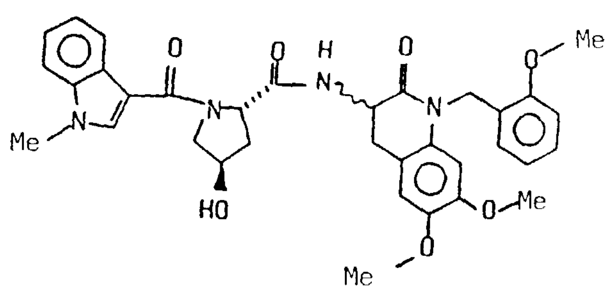

(2S, 4R)R-Form

Die erfindungsgemäßen Verbindungen sind wertvolle Neurokinin (Tachykinin)-Antagonisten, die insbesondere Substanz P-Antagonismus, aber auch Neurokinin A- bzw. Neurokinin-B-antagonistische Eigenschaften besitzen. Sie sind nützlich zur Behandlung von und zur Vorbeugung gegenüber Neurokinin-vermittelten Krankheiten wie Atemwegserkrankungen, z.B. Asthma, Bronchitits, Rhinitis, Husten oder Auswurf sowie von entzündlichen Augenkrankheiten wie beispielsweise Konjunktivitis, von entzündlichen Hautkrankheiten wie beispielsweise Dermatitis und Urticaria, von anderen Entzündungskrankheiten wie Polyarthritis oder Osteoarthritis sowie von Schmerzzuständen und Erbrechen.

Die Erfindung betrifft daher auch die Verwendung der erfindungsgemäßen Verbindungen als Heilmittel und pharmazeutische Zubereitungen, die diese Verbindungen enthalten. Bevorzugt ist die Anwendung am Menschen. Die Applikation der erfindungsgemäßen Verbindungen kann intravenös, subcutan, intramuskulär, intraperitoneal, intranasal, inhalativ, transdermal, gewünschtenfalls durch Iontophorese oder literaturbekannte Enhancer gefördert, und oral erfolgen.

Zur parenteralen Applikation werden die Verbindungen der Formel I oder deren physiologisch veträglichen Salze, eventuell mit den dafür üblichen Substanzen wie Lösungsvermittler, Emulgatoren oder weitere Hilfsstoffe in Lösung, Suspension oder Emulsion gebracht. Als Lösungsmittel kommen z.B. in Frage: Wasser, physiologische Kochsalzlösungen oder Alkohole, z.B. Ethanol, Propandiol oder Glycerin, Zuckerlösungen wie Glucose- oder Mannit-Lösungen oder auch eine Mischung aus verschiedenen Lösungsmitteln.

Außerdem können die Verbindungen durch Implantate, z.B. aus Polylactid, Polyglycolid oder Polyhydroxybuttersäure bzw. intranasale Zubereitungen appliziert werden.

Die erfindungsgemäßen Verbindungen können nach allgemein bekannten Methoden der Amino- und Peptidchemie hergestellt werden, indem man schrittweise die jeweiligen Aminosäuren, beziehungsweise Peptidderivatteilsequenzen, Carbonsäuren und Amine kondensiert und die so erhaltene Verbindung in freier Form oder in Form des gewünschten Salzes isoliert.

Die erfindungsgemäßen Verbindungen können nach allgemein bekannten Methoden der Peptidchemie, wie z.B. in "Houben-Weyl, Methoden der organischen Chemie, Bd. 15/2", beschrieben oder nach der Festphasenpeptidsynthese (z.B. R.C. Sheppard, Int. J. Pept. Prot. Res., 21, 118 [1983]) oder gleichwertigen bekannten Methoden hergestellt werden. Dabei werden die jeweiligen Aminosäuren oder Aminosäureteilsequenzen schrittweise kondensiert und die so erhaltenen Peptide werden in freier Form oder in Form der gewünschten Salze isoliert. Als Aminoschutzgruppen werden die in "Houben-Weyl, Methoden der organischen Chemie, Bd. 15/1" beschriebenen verwendet, wobei in konventionellen Synthesen die Benzyloxycarbonylgruppe (Z) und in Festphasensynthesen die Fluorenylmethoxycarbonylgruppe (Fmoc) bevorzugt wird. Die Seitenkette des Arginins wurde im Fall der konventionellen Synthese durch Protonierung geschützt, im Fall der Festphasensynthese wurde die Mtr-Gruppe verwendet. In der Festphasenpeptidsynthese wurden z.B. auch folgende seitenkettengeschützte Aminosäuren eingesetzt: Lys(Boc), His(Bum), Ser(tBu) und Asp(tBu). Die speziellen Synthesebedingungen sind den nachfolgenden Beispielen zu entnehmen.

Zur Synthese der Verbindungen der allgemeinen Formel I nach der Festphasensynthese werden zunächst die Dipeptidcarbonsäuren synthetisiert, die in Lösung zu den Dipeptidamiden umgesetzt werden. Als Ankergruppen sind folgende geeignet

1. Benzylester (G. Barang, R.B. Merrifield, Peptides 2, 1 (1980) Eds. E. Gross, J. Meienhofer, Academic Press, New York)
2. PAM-Anker (R.B. Merrifield, J. Am. Chem. Soc. 85, 2149 (1966))
3. Wang-Anker (S.-S. Wang, J. Am. Chem. Soc. 95, 1328 (1973))
4. SASRIN-Anker (M. Mergler, R. Tanner, J. Gostuli, P. Grogg, Tetrah. Lett. 29, 4005 (1988)).

Für die Herstellung der Verbindungen der Formel

$$R^1 - C(O) - A^1 - R^5$$

werden die Komponenten $R^1$-COOH, die Aminosäure H-$A^1$-OH und das Amin H-$R^5$ miteinander verknüpft. Es kann wahlweise zuerst die Carbonsäure $R^1$-COOH mit einer geeignet geschützten Form von H-$A^1$-OH gekuppelt und nach Schutzgruppenabspaltung mit dem Amin H-$R^5$ kondensiert werden, oder es kann zuerst die geeignet geschützte Aminosäure H-$A^1$-OH mit H-$R^5$ umgesetzt und nach Deprotektion dieses Produkt mit $R^1$-COOH gekuppelt werden.

Die erfindungsgemäßen Grundkörper der Amine H-$R^5$ können nach an sich bekannten Verfahren gewonnen werden:

Für den Fall, daß H-$R^5$

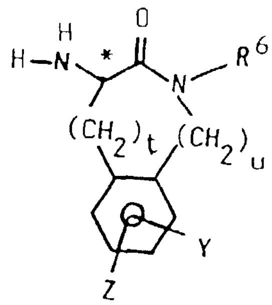

IIa

mit t=1 und u=0 ist und $R^6$, Y und Z wie oben definiert sind, erfolgt die Herstellung nach bekannten Verfahren wie von A.L. Davis et al., J. Med. Chem. 18, 752 (1975) oder H. Merz, DE 38 23 576 (C.A. 114 (21), 207 052 m) beschrieben. Die Einführung des Restes $R^6$ in eine Verbindung der allgemeinen Formel XI erfolgt durch Umsetzen mit NaH und Br$R^6$. Diese Umsetzung kann ohne oder mit Verwendung einer Schutzgruppe (Sch) am exocyclischen N ausgeführt werden.

Diese Herstellung kann durch folgendes Reaktionsschema zusammengefaßt werden:

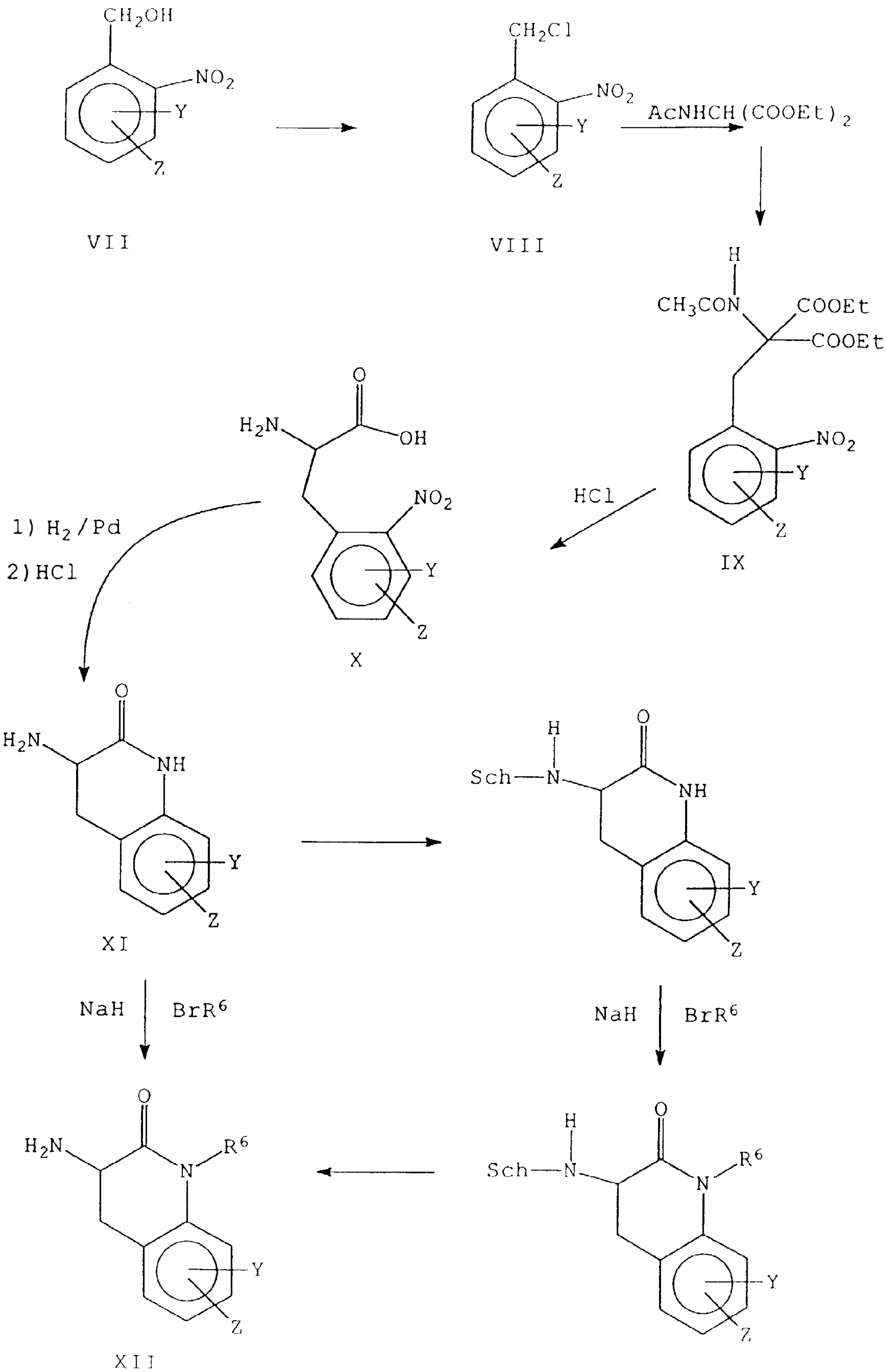


VII

VIII

IX

X

XI

XII

Geeignete Schutzgruppen (Sch) sind basenstabile Schutzgruppen wie beispielsweise die Boc-gruppe.
Zur Herstellung einer Verbindung der allgemeinen Formel XI wird eine Verbindung der allgemeinen Formel X unter Ringschluß reduziert (z.B. analog zu der von A. L. Davis et al. (J. Med. Chem. 9, 826 (1966)) beschrieben mittels Pd-Mohr).
Die Verbindung X kann aus dem entsprechend substituierten 1-Nitrobenzylalkohol (VIII) über die Zwischenstufen VIII und IX (durch Halogenieren mit z.B. $SOCl_2$ und anschließendem Umsetzen mit Acetamidomalonsäurediethylester nach J. Med. Chem. 9, 828 (1966)) hergestellt werden.
Ein Amin H-$R^5$ der allgemeinen Formel IIb

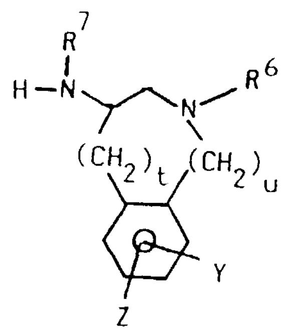

IIb

worin t=1 und u=0 ist und $R^6$, Y und Z wie oben für Formel IIa definiert sind, kann durch Reduktion einer entsprechenden Verbindung IIa mittels z.B. $LiAlH_4$ hergestellt werden.
Für die Herstellung einer Verbindung IIa, worin t=u=0 ist und $R^6$, Y und Z wie oben definiert sind, ist das Verfahren nach A.L. Davis et al., J. Med. Chem. 16, 1043 (1973) geeignet. Dabei wird ausgehend von α-Brom-o-nitrophenylessigsäuremethylester die Phthalimidgruppe eingeführt und nach Abspaltung der Schutzgruppen und der Reduktion der Nitrogruppe cyclisiert zum (substituierten oder unsubstituierten) 3-Amino-2-indolinon:

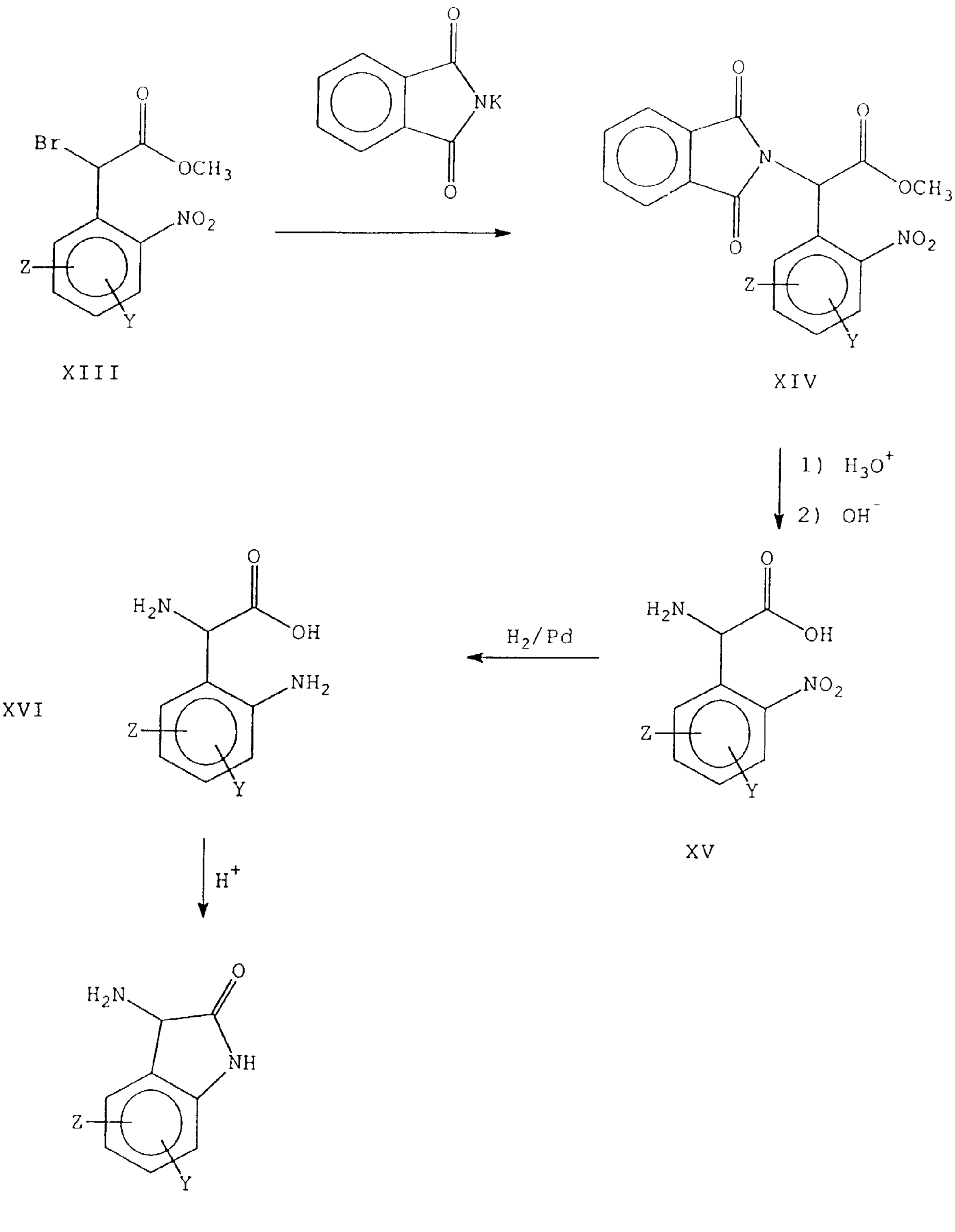


XIII XIV XV XVI XVII

Die Einführung von $R^6$ und Reduktion zur analogen Verbindung der allgemeinen Formel IIb kann wie oben angegeben erfolgen.

Die Herstellung der Verbindung IIa mit t=2, u=0, worin $R^6$, Y und Z wie oben definiert sind, kann, in folgendem Reaktionsschema zusammengefaßt werden:

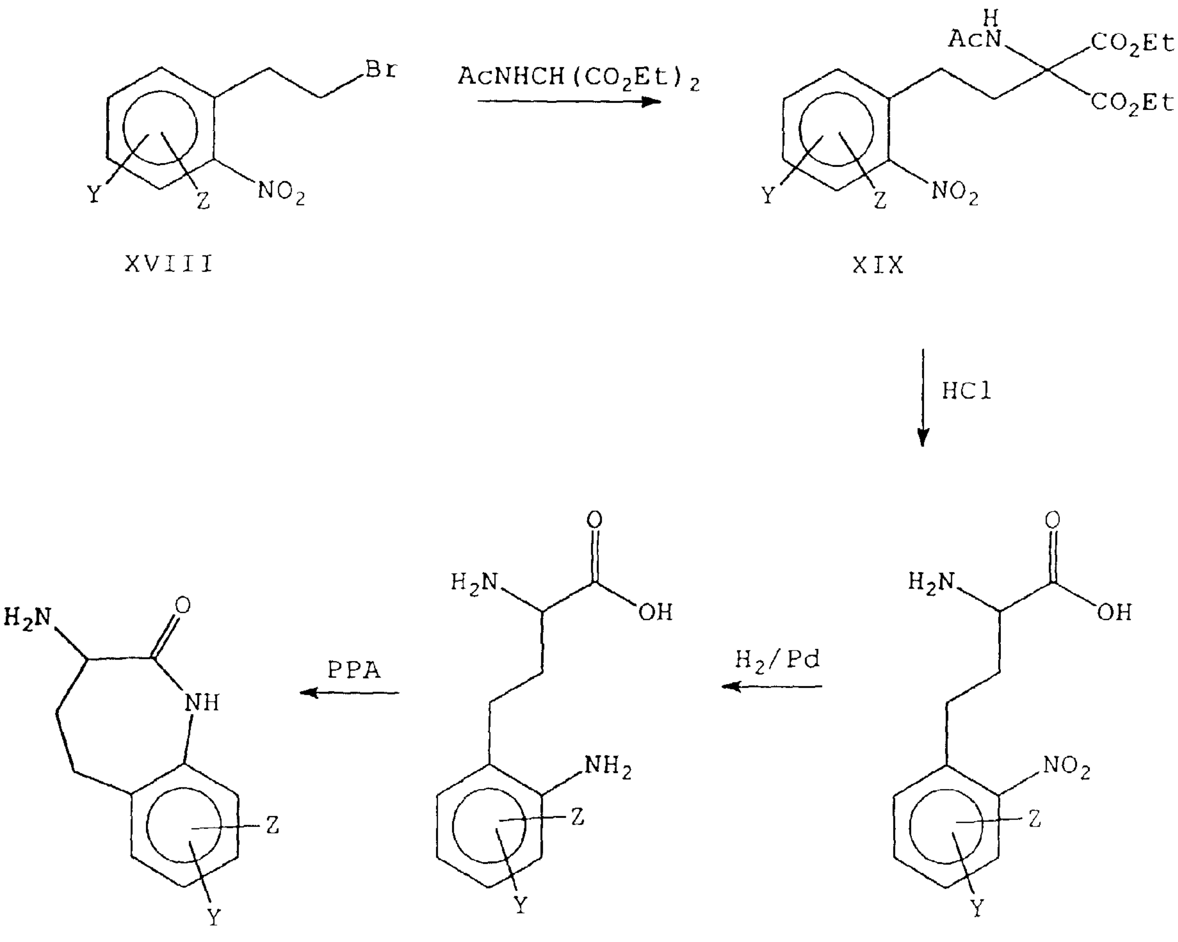


XVIII XIX XX XXI XXII

Die Einführung von $R^6$ u. Reduktion zur analogen Verbindung IIb kann wie oben angegeben erfolgen.

Bei dieser Herstellung kann das entsprechend substituierte 2-(2-Nitrophenyl)-ethylbromid (XVIII) analog zu den oben beschriebenen Verfahren mit Acetamidomalonsäurediethylester zu Verbindung XIX und danach zu XX umgesetzt werden.

Die Reduktion der Verbindung XX zu Verbindung XXI kann z.B. durch Wasserstoff in Gegenwart von Pd-Mohr in einer Lösung aus MeOH und Wasser unter Druck erfolgen. Der Ringschluß zur Herstellung der Verbindung XXII kann durch Polyphosphorsäure unter Rühren und Erhitzen erfolgen.

Die Herstellung der Verbindung IIa mit t=u=1, worin $R^6$, Y und Z wie oben definiert sind, kann auf folgendem Weg erfolgen: Unsubstituiertes oder substituiertes Phthaloyl-phenylalanin wird gekuppelt mit dem Amin $H_2N$-$R^6$ und anschließend in einer Art Pictet-Spengler-Reaktion mit Formaldehyd cyclisiert. Schließlich wird die Phthaloylgruppe abgespalten, z.B. durch Behandlung mit Hydroxylamin:

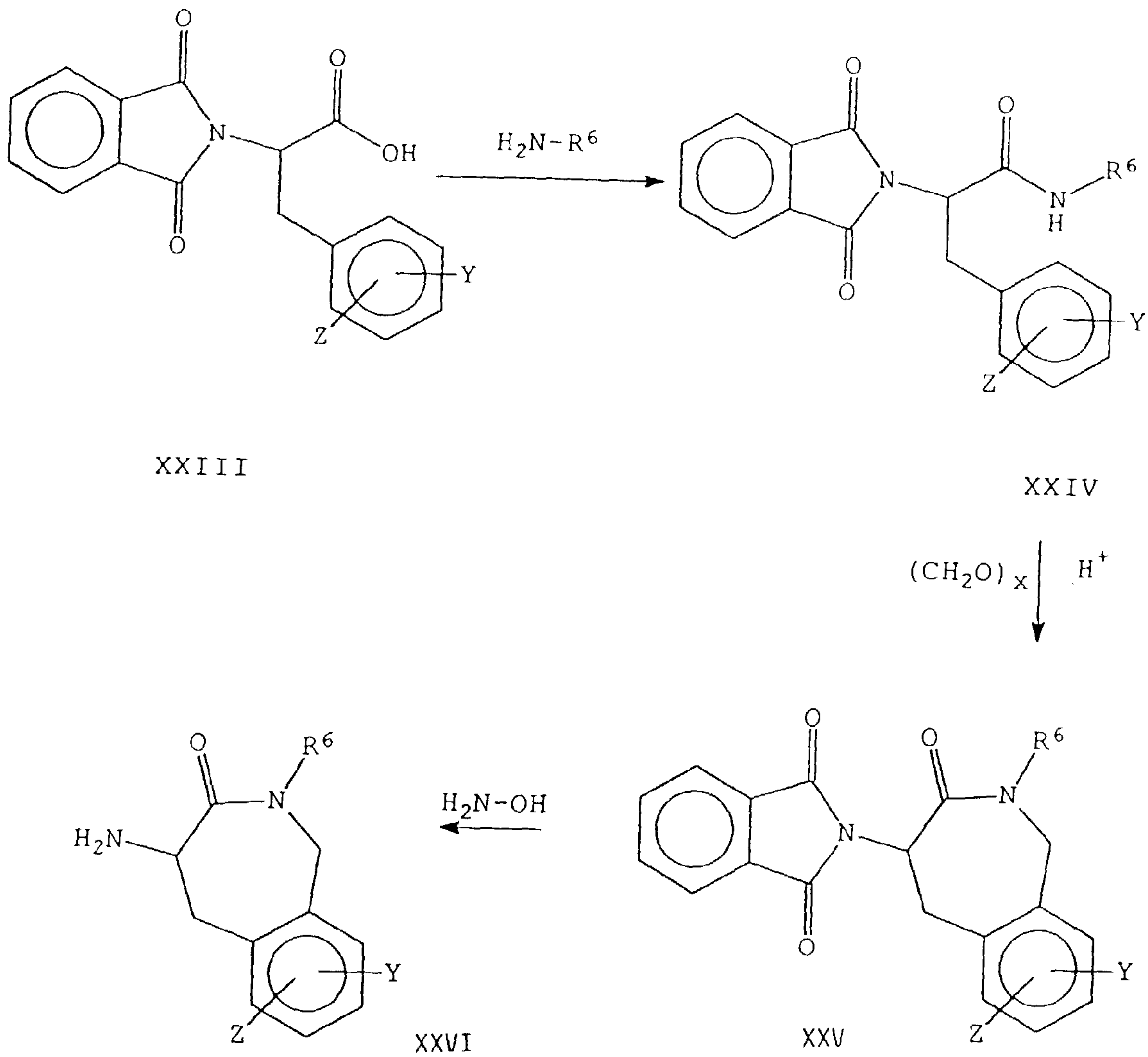


XXIII

XXIV

XXV

XXVI

Die Reduktion zur analogen Verbindung der allgemeinen Formel IIb kann wie oben angegeben erfolgen.

Die Herstellung eines Amins $HR^5$ der allgemeinen Formel IIIa.

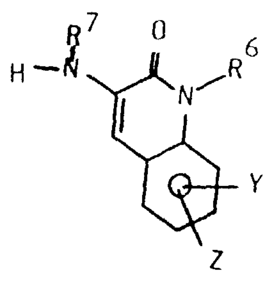

IIIa

worin $R^6$, Y und Z wie oben definiert sind, kann entsprechend G-Leclerc et al., J. Med. Chem. 29, 2427 (1986) erfolgen. Dazu wird substituiertes oder unsubstituiertes 3-Bromchinolin zunächst in das entsprechende N-oxid überführt, dann umgelagert zum Chinolin-2-on und schließlich mit Ammoniak unter Druck (im Bombenrohr) die Aminogruppe eingeführt:

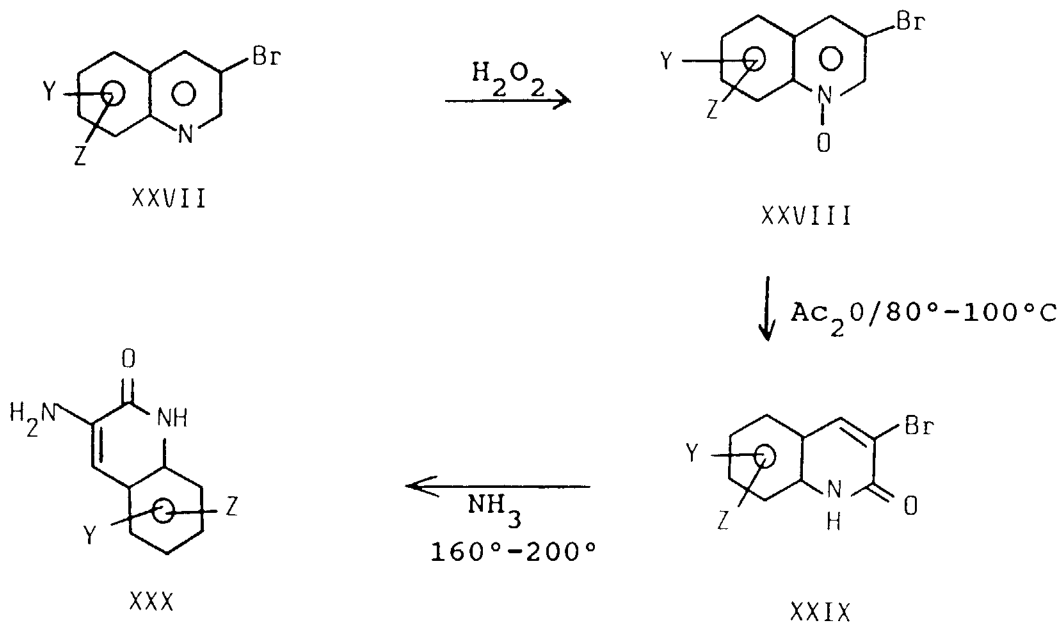


XXVII XXVIII XXIX XXX

Die Einführung des Substituenten $R^6$ kann erfolgen, wie oben für die Verbindung IIa beschrieben.

Die Herstellung einer Verbindung $HR^5$ der allgemeinen Formel IVa,

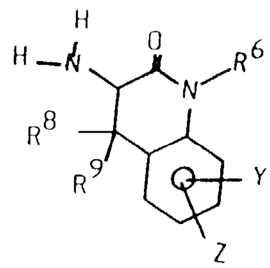

IVa

worin $R^6$ wie oben definiert ist und $R^8$ Hydroxy und $R^9$ Wasserstoff ist, kann nach R. Weichert, Arkiv Kemi 25, 231 (1966) erfolgen. Dabei wird Acetaminomalonsäuremonoethylester mit substituiertem oder unsubstituiertem 2-Nitrobenzaldehyd umgesetzt, anschließend hydrolysiert, die Nitrogruppe reduziert und schließlich cyclisiert:

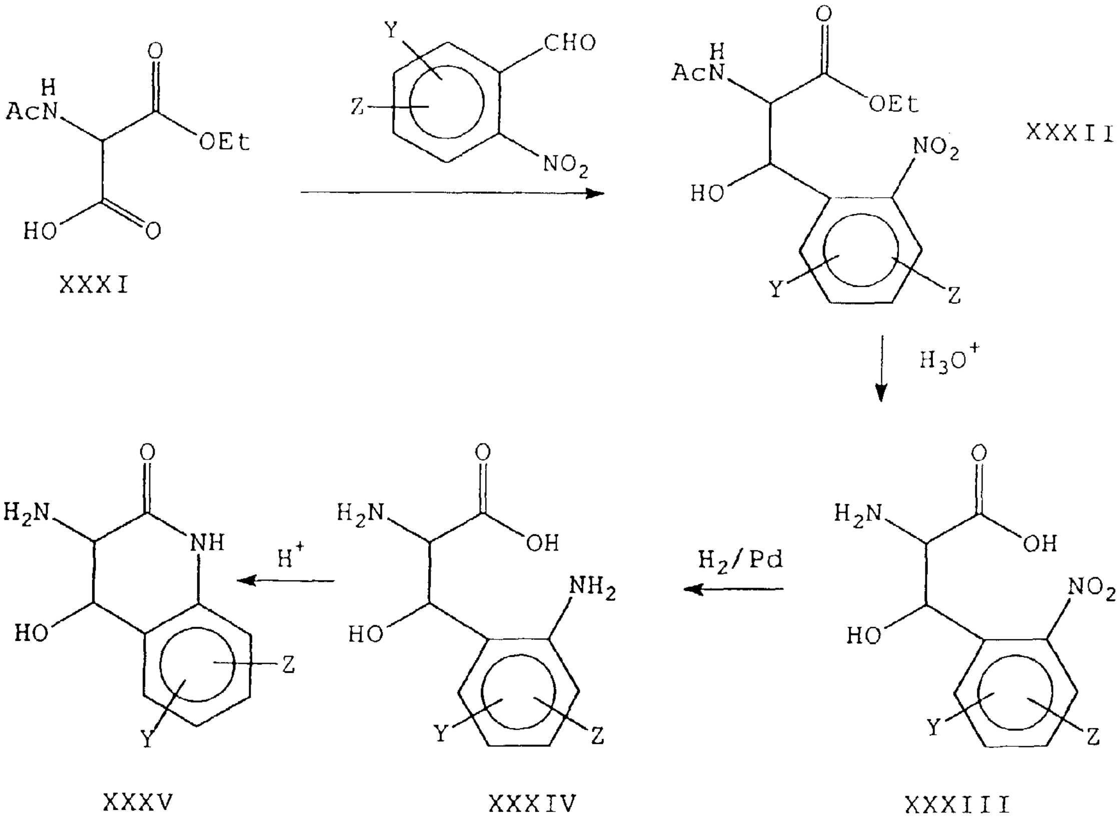


XXXI

XXXII

XXXV

XXXIV

XXXIII

Die Einführung von $R^6$ erfolgt wie oben beschrieben.

Für die Herstellung eine Verbindung IVa, worin $R^9$($C_1$-$C_5$)-Alkoxy, Phenyl-($C_1$-$C_5$)-alkyloxy, Naphthyl-($C_1$-$C_5$)-alkyloxy oder ($C_1$-$C_4$)-alkylcarbonyl ist oder worin $R^8$ und $R^9$ zusammen für Sauerstoff oder $-OCH_2CH_2O-$ stehen, kann die oben angegebene Verbindung IVa, worin $R^8$ Wasserstoff und $R^6$ Hydroxy ist, wie folgt umgesetzt werden:

a) für die Herstellung einer Verbindung IVa, worin $R^9$ Alkyloxy, Phenyl- oder Naphthylalkyloxy ist: Veretherung nach Williamson;

b) für die Herstellung einer Verbindung IVa, worin $R^9$ Alkylcarbonyl ist: Umsetzung mit dem entsprechenden Säureanhydrid;

c) für die Herstellung einer Verbindung IVa, worin $R^8$ und $R^9$ zusammen für Sauerstoff stehen: Oxydation nach z. B. Oppenauer;

d) für die Herstellung zur Verbindung IVa, worin $R^8$ und $R^9$ zusammen $-OCH_2CH_2O-$ sind: Umsetzen der nach (c) erhaltenen Ketoverbindung mit Ethylenglycol.

Für die Herstellung des Amins der allgemeinen Formel H-$R^5$, worin $R^7$ Alkyl ist, werden die Verbindungen der allgemeinen Formeln IIa, IIb, IIIa und IVa alkyliert. Diese Alkylierung kann erfolgen, indem der exocyclische N zunächst durch z.B. Trifluoracetyl geschützt wird, die alkylierung mit z.B. Alkylbromid vorgenommen wird und danach die Schutzgruppe durch z.B. Hydrolyse abgespalten wird.

Pharmazeutische Zubereitungen:

Injektionslösung

| | | | |
|---|---|---|---|
| 200 mg | Wirksubstanz * | | |
| 1,2 mg | Monokaliumdihydrogenphosphat = $KH_2PO_4$ | } | (Puffer) |
| 0,2 mg | Dinatriumhydrogenphosphat = $NaH_2PO_4 \cdot 2H_2O$ | } | (Puffer) |
| 94 mg | Natriumchlorid | } | (Isotonans) |
| oder | | | |
| 520 mg | Glucose | } | (Isotonans) |
| 4 mg | Albumin | | (Proteasenschutz) |
| q.s. | Natronlauge | } | ad pH 6 |
| q.s. | Salzsäure | } | ad pH 6 |
| ad 10 ml | Wasser für Injektionszwecke | | |

Injektionslösung

| | | | |
|---|---|---|---|
| 200 mg | Wirksubstanz* | | |
| 94 mg | Natriumchlorid | | |
| oder | | | |
| 520 mg | Glucose | | |
| 4 mg | Albumin | | |
| q.s. | Natronlauge | } | ad pH 9 |
| q.s. | Salzsäure | } | ad pH 9 |
| ad 10 ml | Wasser für Injektionszwecke | | |

Lyophilisat

| | |
|---|---|
| 200 mg | Wirksubstanz* |
| 520 mg | Mannit (Isotonans/Gerüstbildner) |
| 4 mg | Albumin |

Lösungsmittel 1 für Lyophilisat

| | | |
|---|---|---|
| 10 | ml | Wasser für Injektionszwecke |

Lösungsmittel 2 für Lyophilisat

| | | |
|---|---|---|
| 20 | mg | Polysorbat® 80 = Twen® 80 (oberflächenaktiver Stoff) |
| 10 | ml | Wasser für Injektionszwecke |

* Wirksubstanz: erfindungsgemäße Verbindungen, z.B. die des Beispiels 1

Dosis für Mensch von 67 kg: 1 bis 500 mg

Beispiel 1

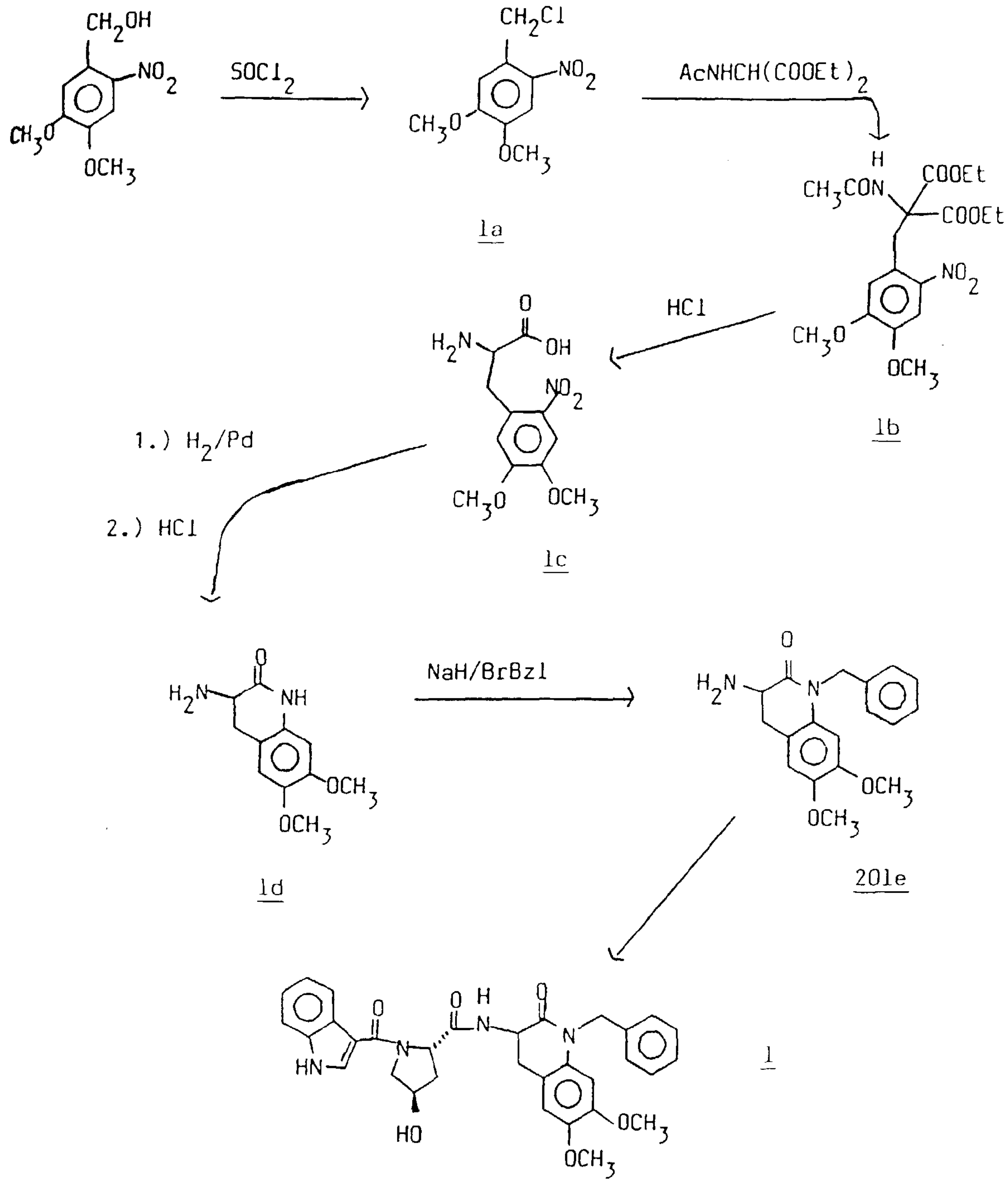


1a

1b

1c

1d

201e

1

Herstellung von 1a:

10,7 g 6-Nitroveratrylalkohol wurden in 20 ml absolutem $SOCl_2$ und 2,7 ml absolutem Pyridin suspendiert, zum Sieden erhitzt und während 1/2h eine Mischung von 4 ml Thionylchlorid und 2 ml $CH_2Cl_2$ zugetropft. Es wurde noch 1h unter Rückfluß gekocht, dann abgekühlt und das Reaktionsgemisch in eine Mischung aus 20 g Eis und 20 g Wasser eingerührt. Die organische Phase wurde gründlich mit Wasser und $NaHCO_3$-Lösung gewaschen, mit $Na_2SO_4$ getrocknet und am Rotationsverdampfer eingeengt. Man erhielt 11,4 g 1a als dunkelbraunes Öl (Ausbeute 98%).

Herstellung von 1b:

11,2 g 1a wurden, wie in J. Med. Chem. 9, 828 (1966) beschrieben, mit 10,5 g Acetamidomalonsäurediethylester umgesetzt, wobei 16,2 g 1b in Form gelber Kristalle erhalten wurden (Ausbeute 81 %). Fp.: 176-178°C.

Herstellung von 1c:

In Anlehnung an die Vorschrift von A.L. Davis (J. Med. Chem. 9, 828, (1966)) wurden 16 g 1b mit 120 ml konzentierter Salzsäure hydrolysiert, wobei zunächst 1c.HCl erhalten wurde. Dieses wurde mittels Ammoniak in die freie Aminosäure 201c überführt, wovon 7,4 g in Form grünlicher Kristalle erhalten wurden (Ausbeute 71 %). Fp.: ca. 207°C (Zers.).

Herstellung von 1d:

5,4 g 1c wurden, wie von A.L. Davis et al. (J. Med. Chem. 9, 828 (1966)) beschrieben, mittels 0,6 g Pd-Mohr hydriert. Die erhaltene Aminoverbindung wurde zusammen mit 68 ml Ethanol und 12 ml konzentrierter Salzsäure 1/2h unter Rühren am Rückfluß gekocht. Nach dem Abkühlen wurde mit 26 ml Ether versetzt, abgesaugt und der Niederschlag mit eiskaltem Ethanol und Ether gewaschen und bei 80°C getrocknet. Man erhielt 3,3 g 1d.HCl in Form einer hellgrauen Festsubstanz (Ausbeute 63 %). Fp.: ca. 296°C (Zers.).

Herstellung von 1e:

1,3 g 1d.HCl wurden in 15 ml DMF gelöst, mit 0,42 g NaH-Dispersion (60 %ig in Mineralöl) versetzt, 1/2h bei Raumtemperatur gerührt, dann 0,66 ml Benzylbromid langsam zugetropft und 1 h bei Raumtemperatur gerührt. Das Reaktionsgemisch wurde in ca. 200 ml Wasser eingerührt und 2x mit Essigester extrahiert. Die vereinten Essigesterphasen wurden filtriert und eingeengt, in Ether aufgenommen und mit etherischer HCl versetzt und erneut eingeengt. Der feste Rückstand wurde mit Ether angerührt, abgesaugt und im Exsikkator über KOH getrocknet, wobei 1,25 g 1e. HCl in Form einer beigen Festsubstanz gewonnen wurden (Ausbeute 72 %).
Fp.: 98-116°C.

Herstellung von ( 1):

a) Synthese von (2S, 4R)-N-(indol-3-yl-carbonyl)-4-hydroxy-prolin: 9,2 g (2S, 4R)-Hydroxyprolin, 105 ml $CH_2Cl_2$ und 35,4 ml Chlortrimethylsilan wurden vereint, 1 h bei Raumtemperatur gerührt, dann 1 h unter Rückfluß gekocht, innerhalb von 15 min 39 ml Triethylamin zugetropft und weitere 15 min unter Rückfluß gekocht. Die Reaktionsmischung wurde auf -70°C gekühlt und innerhalb 40 min eine Lösung aus Indol-3-carbonsäurechlorid in 100 ml $CH_2Cl_2$ und 50 ml Essigester zugetropft. Man rührte weitere 20 min bei -70°C und ließ innerhalb 1,5 h auf Raumtemperatur erwärmen. Unter Eiskühlung wurden nacheinander 190 ml Wasser und 25 ml 2N Salzsäure zugesetzt, weitere 45 min bei Raumtemperatur gerührt und über Nacht bei Raumtemperatur stehen gelassen. Der ausgefallene Niederschlag wurde abgesaugt, nacheinander mit $CH_2Cl_2$, Wasser, Essigester und Ether gewaschen und im Exsikkator getrocknet. Man erhielt 15,5 g (2S, 4R)-N-(indol-3-ylcarbonyl)-4-hydroxyprolin in Form beiger Kristalle (Ausbeute 91 %).
$[\alpha]_D^{20}$ (MeoH) = -136,4°C.

b) Kupplung von (2S, 4R)-N-(indol-3-ylcarbonyl)-4-hydroxyprolin mit le: 0,41 g (2S, 4R)-N-(indol-3-ylcarbonyl)-4-hydroxyprolin wurden in 150 ml DMF gelöst, mit 0,81 g 1e.HCl versetzt und mit Triethylamin auf pH 8,5 gestellt. Es wurde auf -20°C abgekühlt, mit 0,4 ml DPPA (Diphenylphosphorylazid) versetzt, 3 Tage im Tiefkühlschrank bei -25°C und 1 Tag bei 8 °C im Kühlschrank belassen. Die Reaktionsmischung wurde am Rotationsverdampfer eingeengt, in $CH_2Cl_2$ aufgenommen und nacheinander mit verdünnter Salzsäure, gesättigter $NaHCO_3$-Lösung und 10 %iger NaCl-Lösung ausgeschüttelt. Die organische Phase wurde filtriert, eingeengt und der Rückstand über Kieselgel chromatographiert. Aus den einheitlichen Fraktionen des Eluats wurden 0,48 g ( 1) als weiße Festsubstanz gewonnen (Ausbeute 56 %).
Fp.: 128-142°C (Zers.).
$[\alpha]_D^{20}$ (MeoH) = -103,7°C.

Die Substanz stellt ein ca.

1:1-Diastereomerengemisch dar, worin im Aminteil $R^2$ zum einen R-, zum anderen S-Konfiguration vorliegt.

Beispiel 2, (2S, 4R)S-Form und Beispiel 3, (2S, 4R)R-Form

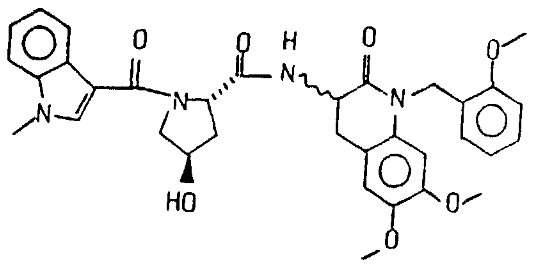

Die Synthese wurde durchgeführt wie unter Beispiel 1 beschrieben, mit dem Unterschied, daß 1-Methyl-indol-3-carbonsäurechlorid anstelle von Indol-3-carbonsäurechlorid eingesetzt wurde und 2-Methoxybenzylchlorid anstelle von Benzylbromid. Schließlich wurde das Substanzgemisch der letzten Reaktionsstufe an einer Kieselgelsäule mit Essigester/MeOH (4:1) in die Diastereomeren aufgetrennt.

Die schneller wandernde Substanz ( 2) ($R_f$ = 0,44) konnte in einer Menge von 0,82 g gewonnen werden (Ausbeute 26 %), wobei es sich wahrscheinlich um die (2S, 4R)S-Form handelt.
Fp.: 201-208°C.
$[\alpha]_D^{20}$ (DMSO) = -135°C.

Von der langsamer wandernden Substanz ( 3) ($R_f$ = 0,38) wurden 0,31 g erhalten (Ausbeute 10 %); hierbei handelt es sich wahrscheinlich um die (2S, 4R) R-Form.
Fp.: 123-133°C.
$[\alpha]_D^{20}$ (MeOH) = -24,2°C.

Beispiel 14

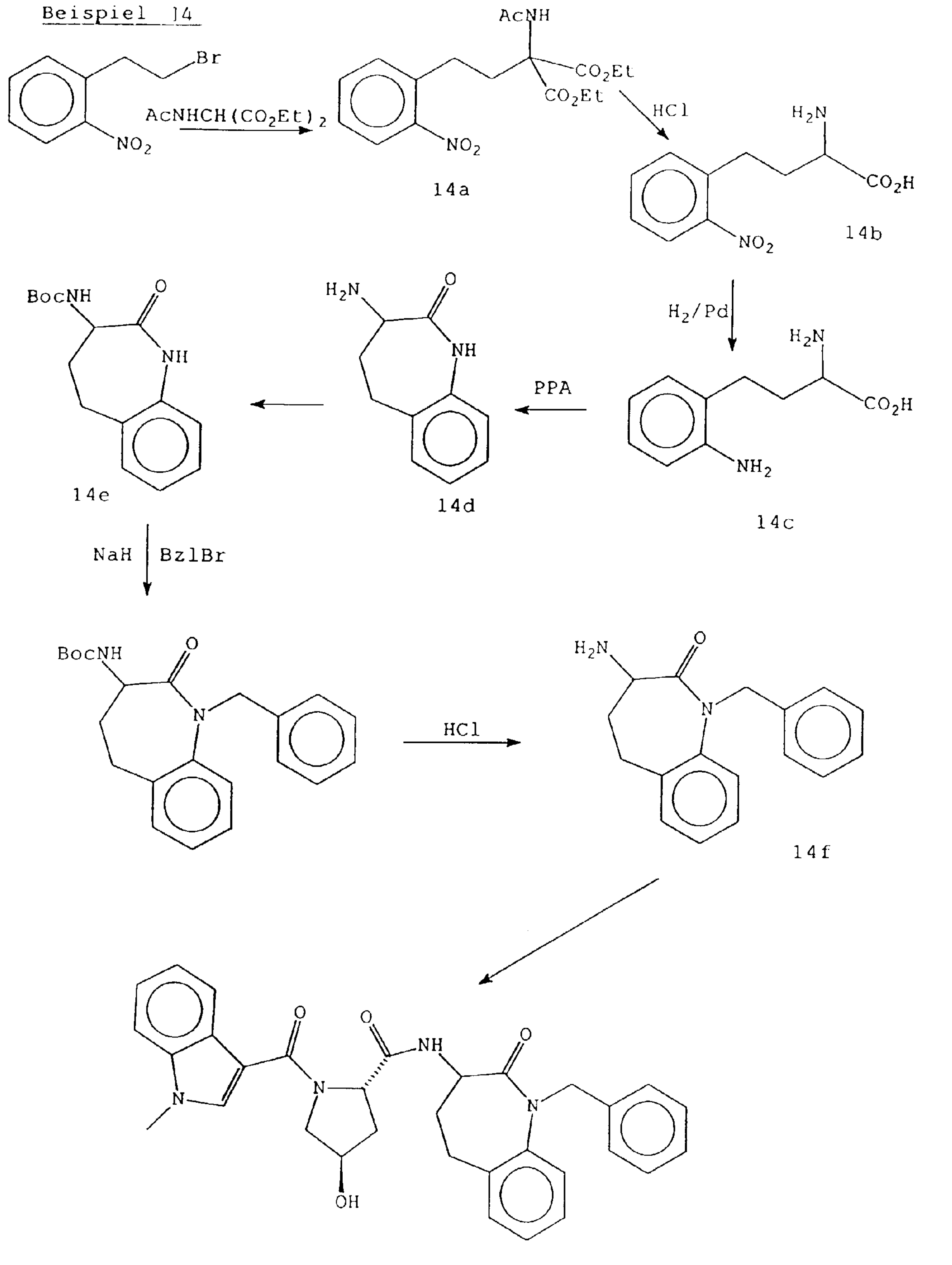


14

Herstellung von 14a

22,5 g 2-(2-Nitrophenyl)-ethylbromid wurden, wie für die Herstellung von 1b beschrieben, umgesetzt mit 23,4

g Acetamidomalonsäurediethylester, wobei 29,6 g 14a erhalten wurden (Ausbeute 83 %).

Herstellung von 14b

Analog zur Herstellung von 1c wurden 29,6 g 14a mit 315 ml konz. HCl hydrolysiert, wobei 16,5 g 14b in Form hellgelber Kristalle isoliert wurden (Ausbeute 91 %).

Herstellung von 14c

16,2 g 14b wurden mittels 3,25 g Pd-Mohr in einer Lösung aus 600 ml MeOH und 200 ml Wasser unter Druck hydriert. Dabei wurden 12,9 g 14c in Form eines hellgelben Pulvers gewonnen (Ausbeute 92 %).

Herstellung von 14d

12,8 g 14c wurden mit 100 g Polyphosphorsäure vereint und unter Rühren für 3 h auf 120-130°C erhitzt. Nach dem Abkühlen wurde in ca. 400 g Eis eingerührt, mit Ammoniak alkalisiert und mit 3 x 400 ml $CH_2Cl_2$ extrahiert. Die organischen Extrakte wurden eingedampft und der Rückstand mit Ether verrührt und getrocknet. Es wurden 9,4 g 14d in Form beiger Kristalle erhalten (Ausbeute 81 %).

Herstellung von 14e

9,4 g 14d wurden in 100 ml THF und 50 ml Wasser gelöst, mit 12,8 g $(Boc)_2O$ versetzt und 30 min bei Raumtemperatur gerührt. Am Rotationsverdampfer wurde das THF abgezogen und der entstehende Niederschlag abgesaugt und getrocknet. Man erhielt 14,5 g 14e in Form hellbeiger Kristalle (Ausbeute 98 %).

Herstellung von 14f

1,9 g 14e wurden in 40 ml DMF gelöst, mit 0,3 g NaH-Dispersion (60% in Öl) versetzt und 45 min bei Raumtemperatur gerührt. Zu der Reaktionsmischung wurde dann innerhalb 10 min eine Lösung von 0,9 ml Benzylbromid in 10 ml THF unter Rühren bei Raumtemperatur zugetropft und weitere 20 min bei Raumtemperatur gerührt. Die Mischung wurde einrotiert und der Rückstand mit 40 ml $CH_2Cl_2$, 4 ml Anisol und 40 ml 4nHCl in Dioxan versetzt und 1 h bei Raumtemperatur belassen. Die Reaktionsmischung wurde am Rotationsverdampfer eingeengt, der Rückstand mit Ether gewaschen und dann in 50 ml Wasser gelöst. Die Lösung wurde 2x ausgeethert, die wäßrige Phase mit 1 M $Na_2CO_3$-Lösung alkalisiert und 2 mal mit Ether extrahiert. Durch Filtration und Eindampfen erhielt man aus dem Etherextrakt 1,63 g 214f in Form eines bräunlichen, zähen Öls (Ausbeute 87 %).

Herstellung von 14

Wie für die Herstellung von 1 beschrieben wurden 1,75 g (2S, 4R)-N-(1-Methyl-indol-3-ylcarbonyl)-4-hydroxyprolin mit 1,62 g 14f gekuppelt. Man erhielt 2,6 g 14 in Form weißer Kristalle (Ausbeute 80 %). Fp.: 118-123°C.

Tabelle 1

Zusammenfassung der Beispiele, die in analoger Weise hergestellt werden können.

Beispiel 1

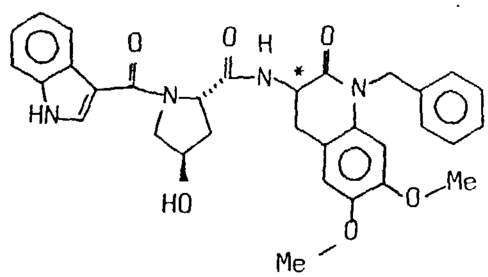

*R/S

Beispiel 2

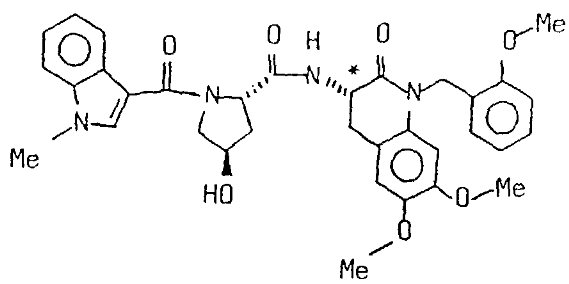

*S

Beispiel 3

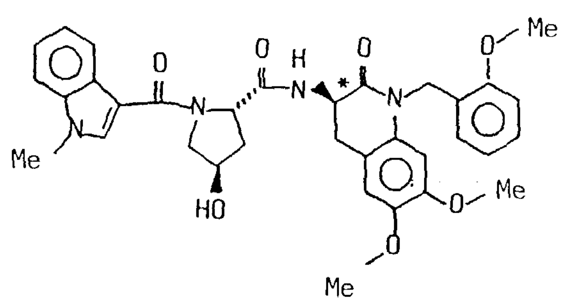

*R

Beispiel 4

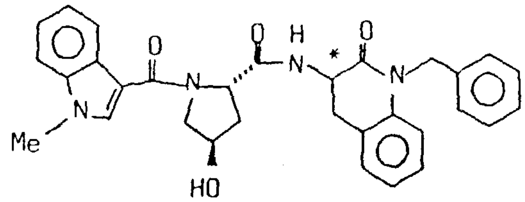

*R/S

Beispiel 5

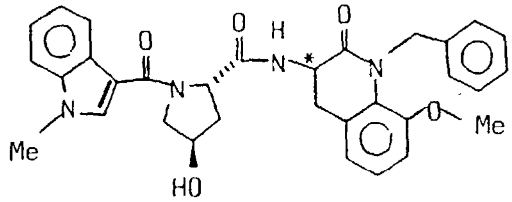

*R/S

Beispiel 6

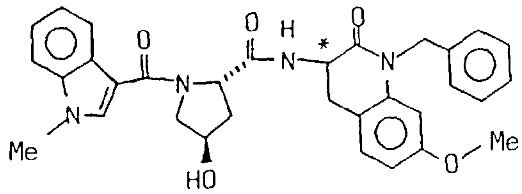

*R/S

Beispiel 7

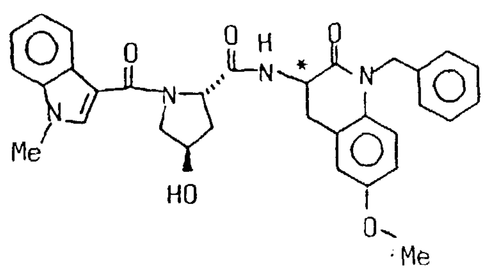

*R/S

Beispiel 8

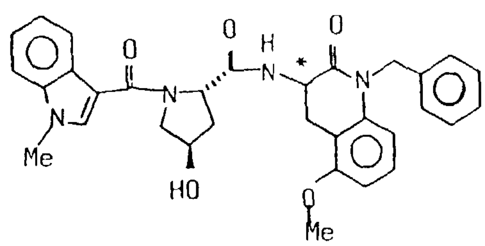

*R/S

Beispiel 9

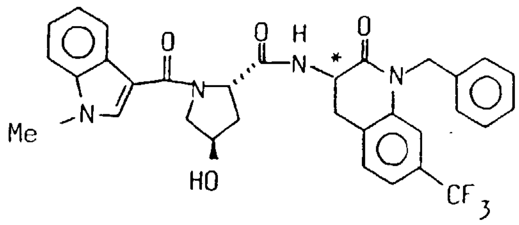

*R/S

Beispiel 10

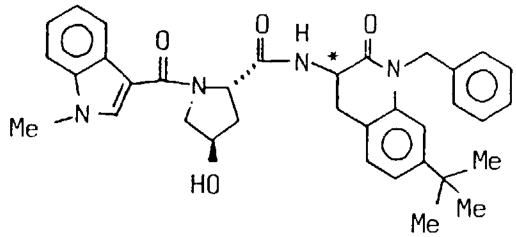

*R/S

Beispiel 11

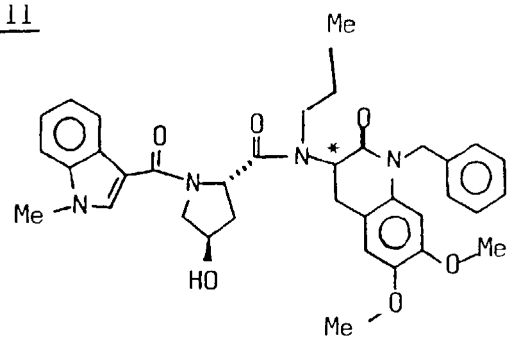

*R/S

Beispiel 12

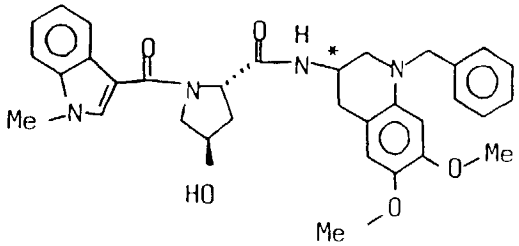

*R/S

Beispiel 13

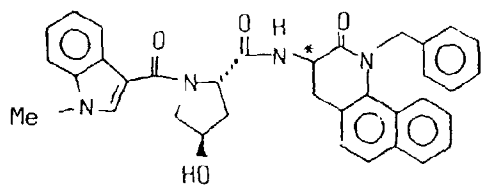

*R/S

Beispiel 14

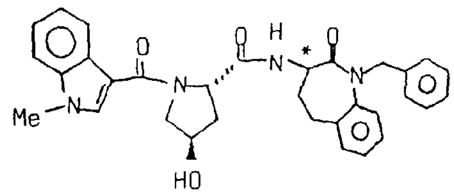

*R/S

Beispiel 15

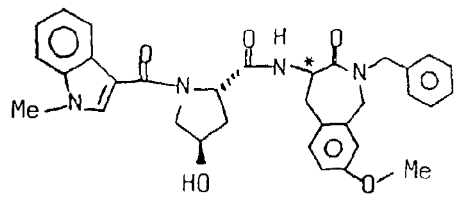

*R/S

Beispiel 16

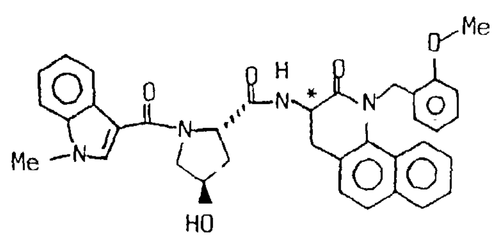

*R/S

Beispiel 17

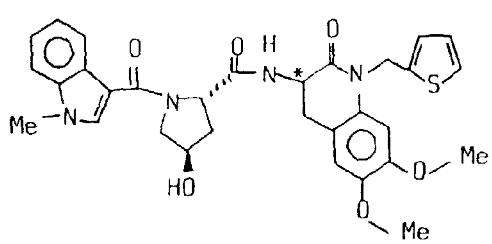

*R/S

Beispiel 18

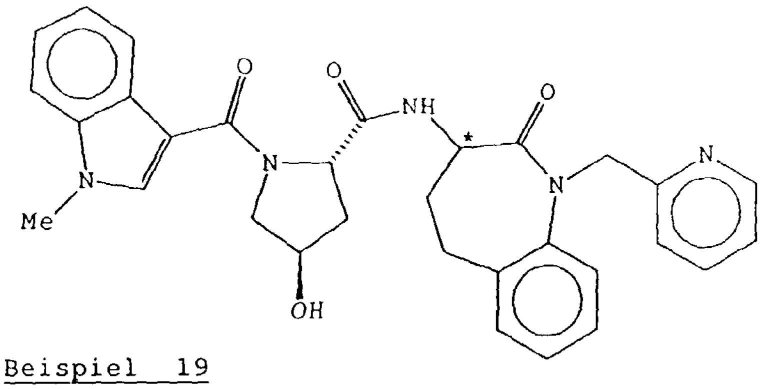

* R/S

Beispiel 19

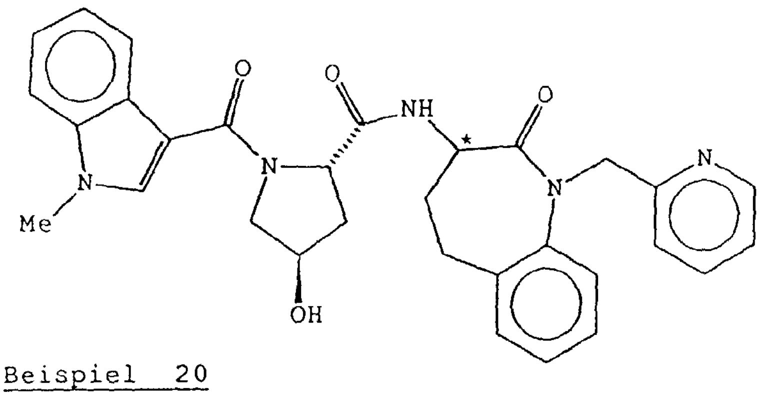

Beispiel 20

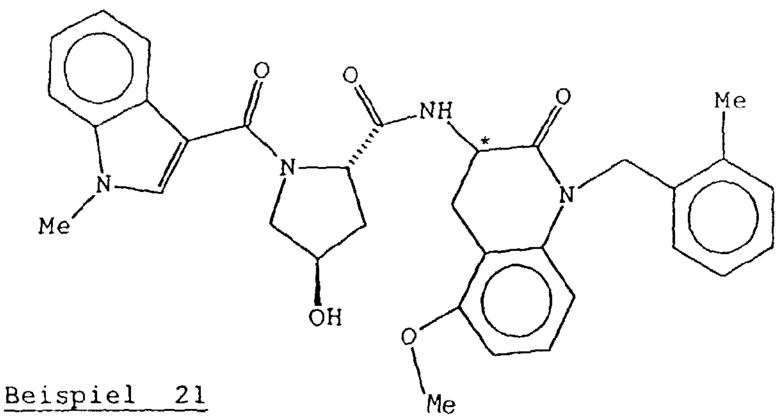

* R/S

Beispiel 21

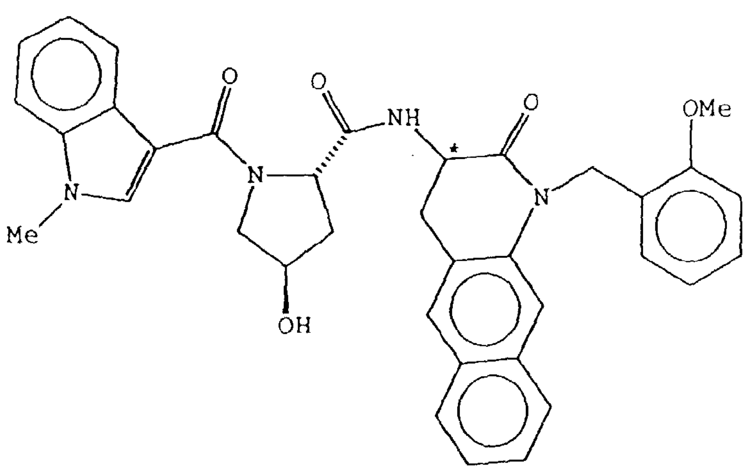

* R/S

Physikalische Daten:

Die Schmelzpunkte wurden an einem Büchi-510-Schmelzpunkt-Gerät gemessen, die Drehwerte an einem Perkin-Elmer-241-Polarimeter.

Beispiel 1

Fp.: 128 - 142°C (Zers.); $[\alpha]_D^{20}$ (MeOH) = -103,7°C

Beispiel 2

Fp.: 201 - 208°C; $[\alpha]_D^{20}$ (DMSO) = -135°C

Beispiel 3

Fp.: 123 - 133°C; $[\alpha]_D^{20}$ (MeOH) = -24,2°C

Beispiel 4

Fp.: ab 128°C (Zers.); $[\alpha]_D^{20}$ (MeOH) = -74,2°C

Beispiel 5

Fp.: ab 105°C (Zers.); $[\alpha]_D^{20}$ (MeOH) = -93,3°C

Beispiel 6

Fp.: 160 - 165°C; $[\alpha]_D^{20}$ (MeOH) = -135°C

Beispiel 9

Fp.: 220 - 250°C; $[\alpha]_D^{20}$ (MeOH) = -77,2°C

Beispiel 10

Fp.: 220 - 235°C; $[\alpha]_D^{20}$ (MeOH) = -101,2°C

Beispiel 14

Fp.: 118 - 123°C; $[\alpha]_D^{20}$ (MeOH) = -94,2°C

Beispiel 18

Fp.: 137 - 142°C; $[\alpha]_D^{20}$ (MeOH) = -95,6°C

Beispiel 19

Fp.: 120 - 126°C; $[\alpha]_D^{20}$ (MeOH) = +27°C

Beispiel 20

Fp.: 96 - 103°C; $[\alpha]_D^{20}$ (MeOH) = -92,8°C

Beispiel 21

Fp.: 230 - 242°C; $[\alpha]_D^{20}$ (DMSO) = -65,8°C

Tabelle 2

Beispiele 22 - 31

Beispiel 22

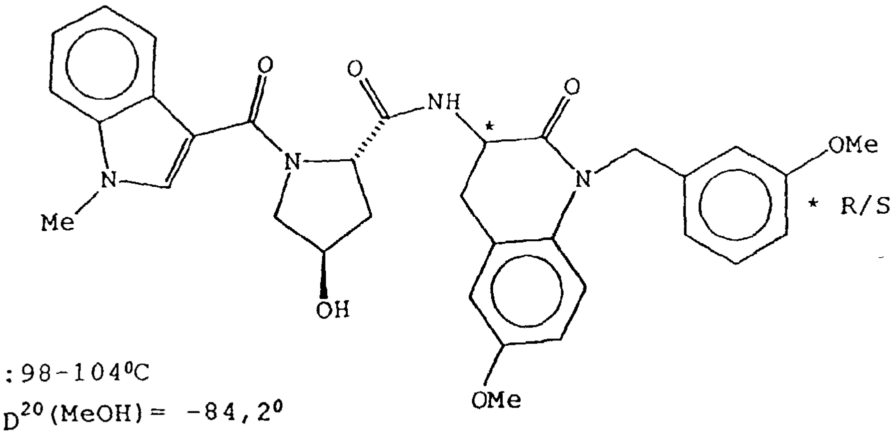

* R/S

Fp.: 98-104°C

[α]$_D^{20}$(MeOH) = -84,2°

Beispiel 23

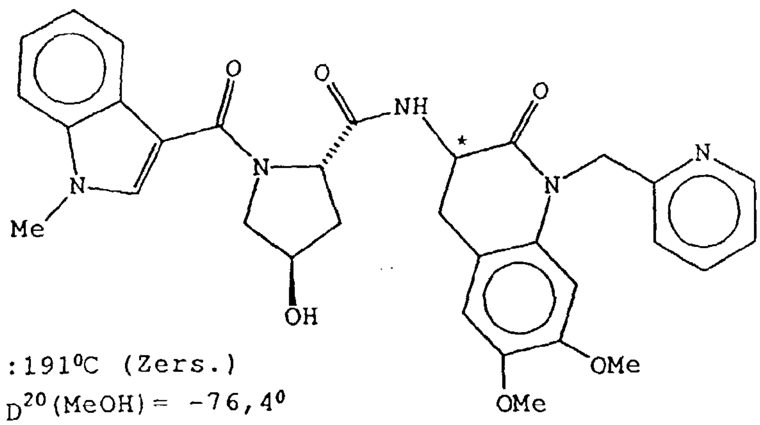

.HCl * R/S

Fp.: 191°C (Zers.)

[α]$_D^{20}$(MeOH) = -76,4°

Beispiel 24

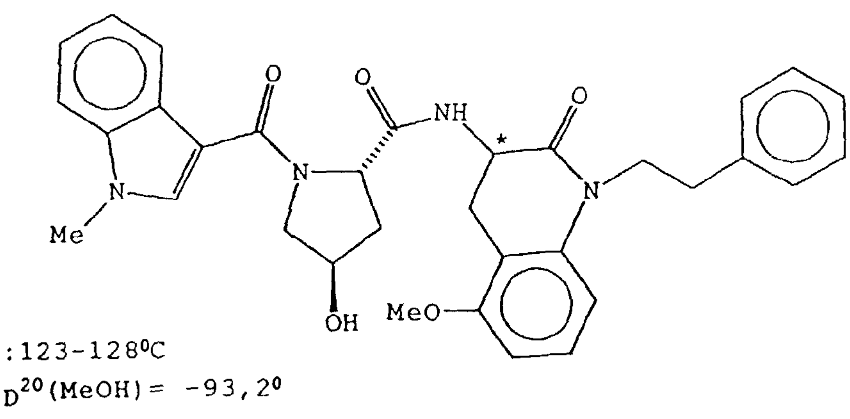

* R/S

Fp.: 123-128°C

[α]$_D^{20}$(MeOH) = -93,2°

Beispiel 25

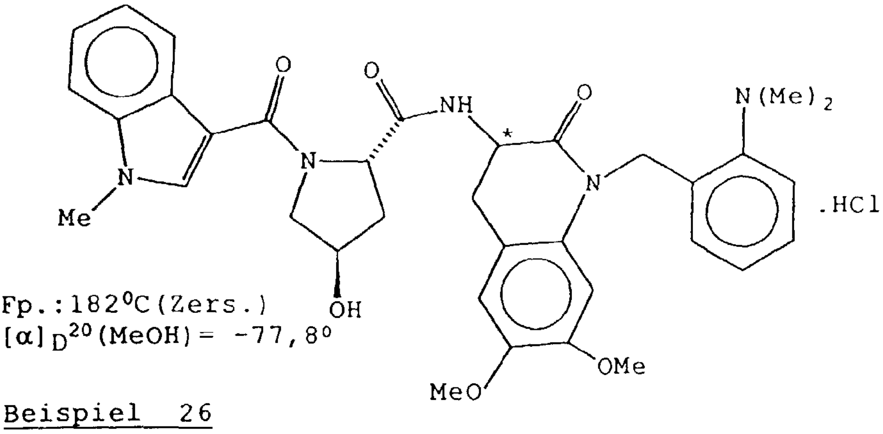

* R/S

Fp.: 182°C (Zers.)
[α]$_D^{20}$ (MeOH) = -77,8°

Beispiel 26

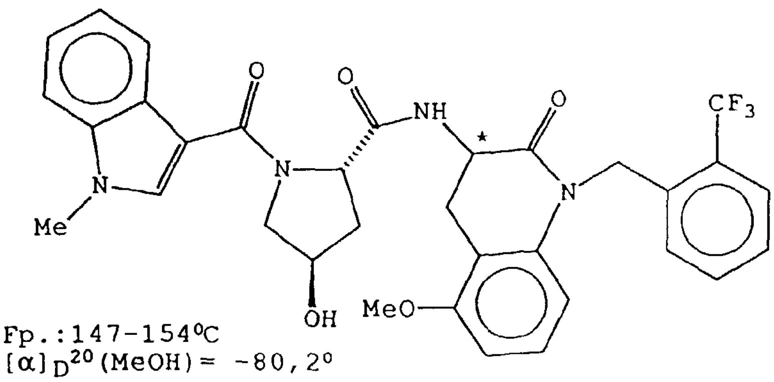

* R/S

Fp.: 147-154°C
[α]$_D^{20}$ (MeOH) = -80,2°

Beispiel 27

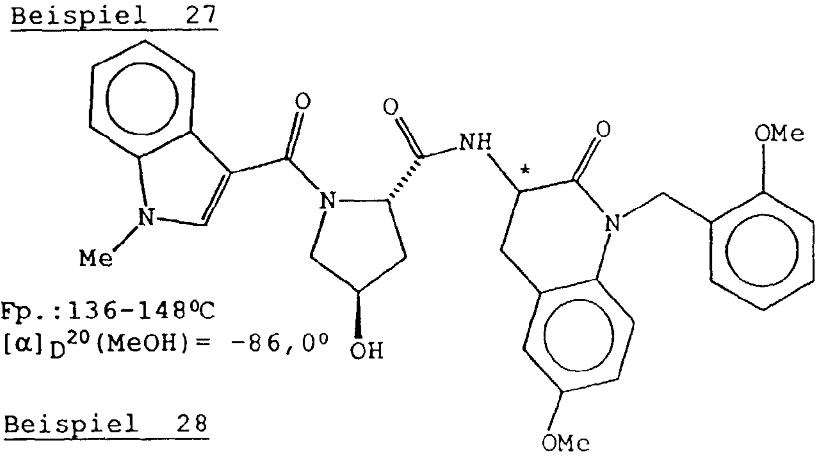

* R/S

Fp.: 136-148°C
[α]$_D^{20}$ (MeOH) = -86,0°

Beispiel 28

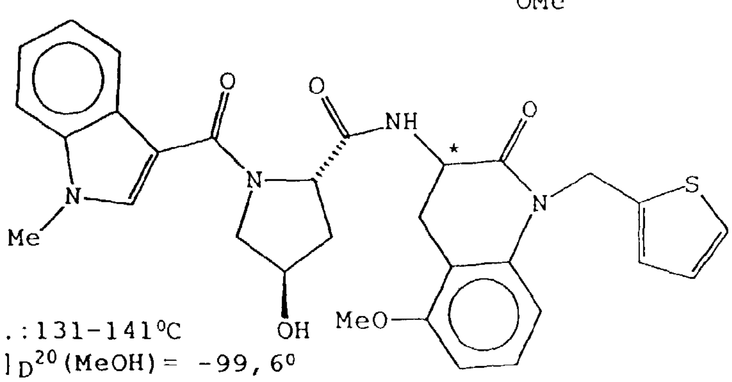

* R/S

Fp.: 131-141°C
[α]$_D^{20}$ (MeOH) = -99,6°

Beispiel 29

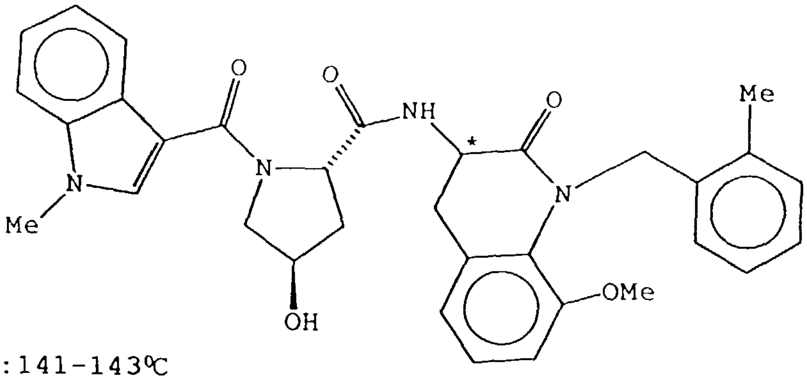

* R/S

Fp.:141-143°C
[α]$_D^{20}$(MeOH)= -106,6°

Beispiel 30

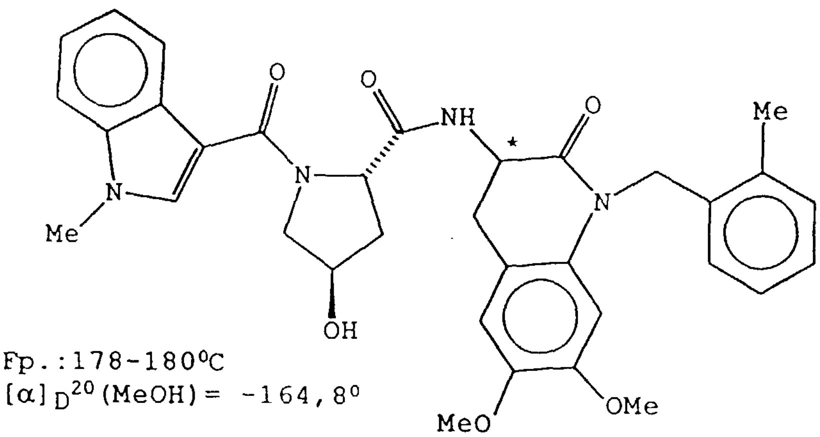

* S

Fp.:178-180°C
[α]$_D^{20}$(MeOH)= -164,8°

Beispiel 31

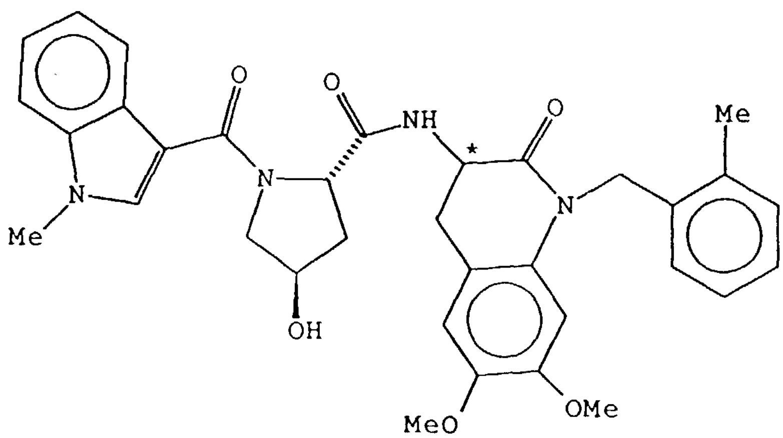

* R

Fp.:110-120°C(Zers.)
[α]$_D^{20}$(MeOH)= -19,4°

## Patentansprüche

1. Aminosäurederivat der allgemeinen Formel I

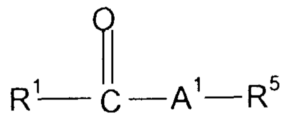

(I)

oder dessen pharmazeutisch annehmbares Salz, worin

$R^1$ Vinyl, Aryl, Heteroaryl, Aralkyl, Heteroaralkyl, Arylvinyl, Heteroarylvinyl, Aryloxyalkyl, Arylalkyloxy, ($C_3$-$C_8$) Cycloalkyl, ($C_3$-$C_8$)Cycloalkylalkyl, unsubsituiertes oder durch 1-3 Methylgruppen substituiertes Bicycloheptyl oder Bicycloheptylalkyl, Adamantyl, Adamantylalkyl, Dekalin, Dekalinalkyl, Tetralin, Tetralinalkyl, Diphenylalkyl, Di(arylalkyl)aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl, Hydroxy, Nitro, Alkylcarbonyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D-oder L-Threonin (Thr), D- oder L-alloThreonin, D- oder L-Cystein (Cys), D- oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D- oder L-Didehydroprolin (ΔPro) wie beispielsweise 3,4-Didehydroprolin (Δ (3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin (Pro($NH_2$)) wie beispielsweise 3-Aminoprolin (Pro(3$NH_2$)) und 4-Aminoprolin (Pro(4$NH_2$)), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), Tpr($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können;

$R^5$ für ein Amin der Formel

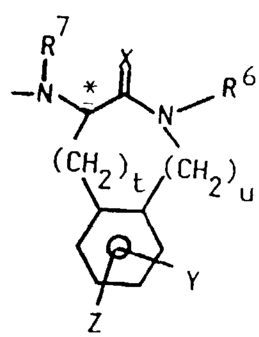

oder

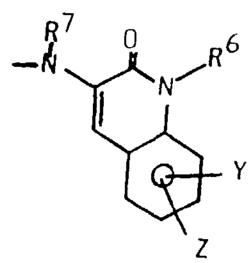

II

III

steht, worin

$R^6$ für Aralkyl, Diarylalkyl (in diesen Gruppen ist Aryl Phenyl oder Naphthyl und Alkyl ($C_1$-$C_5$)Alkyl)), Heteroaryl-($C_1$-$C_5$)-alkyl (worin Heteroaryl 2-,3- oder 4-Pyridyl oder 2- oder 3-Thienyl ist), Phenylamino-($C_1$-$C_5$)-alkyl, Nephthylamino-($C_1$-$C_5$) -alkyl oder N-Phenylalkylpiperidinyl (worin die angeführten Phenylgruppen unsubstituiert sind oder 1, 2 oder 3 Substituenten enthalten, die unabhängig voneinander ($C_1$-$C_5$)-Alkyl, vorzugsweise Methyl, ($C_1$-$C_5$)-Alkoxy, vorzugsweise Methoxy, Dimethylamin, Halogen, Trifluormethyl, -CN oder $OCF_3$ sind) steht;

$R_7$ für Wasserstoff oder ($C_1$-$C_5$)-Alkyl steht;

X für 0 oder $H_2$ steht;

Y und Z unabhängig voneinander für Wasserstoff ($C_1$-$C_5$)-Alkyl, ($C_1$-$C_5$)-Alkyloxy, Benzyloxy (worin die Phenylgruppe unsubstituiert ist oder 1, 2 oder 3 Substituenten enthält, die unabhängig voneinander ($C_1$-$C_5$)-Alkyl, vorzugsweise Methyl, ($C_1$-$C_5$)-Alkoxy, vorzugsweise Methoxy, Dimethylamin, Halogen, Trifluormethyl, -CN oder $OCF_3$ sind), $OCF_3$, Halogen, $CF_3$, CN, $CH_2NH_2$, $CONH_2$, N-($C_1$-$C_5$-Alkyl)$_2$, NH-($C_1$-$C_4$)-alkylcarbonyl, N-($C_1$-$C_5$)-Alkyl-N-($C_1$-$C_4$)-alkylcarbonyl, $NH_2$ oder NH-($C_1$-$C_5$)-Alkyl stehen oder wenn Y und Z vicinal zueinander stehen, diese zusammen -$OCH_2O$-, $OCH_2CH_2O$- oder $(CH)_4$ bedeuten;

t und u eine der folgenden Bedeutungen haben

(a) t und u null
(b) t eins und u null
(c) t und u je eins
(d) t zwei und u null;

und wenn t eins und u null ist, $R^5$ auch für ein Amin der Formel IV

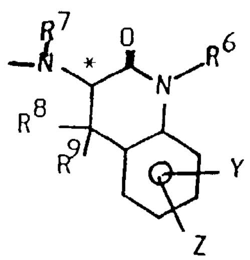

IV

steht, worin

$R^6$, $R^7$, Y und Z die oben genannte Bedeutung haben und

$R^8$ für Wasserstoff steht und $R^9$ für Hydroxy, ($C_1$-$C_5$)-Alkoxy, Phenyl-($C_1$-$C_5$)-alkyloxy, Naphthyl-($C_1$-$C_5$) -alkyloxy oder ($C_1$-$C_4$)-Alkylcarbonyl, oder worin

$R^8$ und $R^9$ zusammen für Sauerstoff oder -$OCH_2CH_2O$- stehen;

und die Chiralität an C* R oder S bedeuten kann.

**2.** Verbindung nach Anspruch 1, worin

$R^1$ Vinyl, Aryl, Heteroaryl, Aralkyl, Heteroaralkyl, Arylvinyl, Heteroarylvinyl, Aryloxyalkyl, Arylalkyloxy, Di(arylalkyl)aminoalkyl oder Arylalkylaminoalkyl (worin Aryl für Phenyl, 1-, 2- oder 3-fach substituiertes Phenyl oder Naphthyl steht; die Substituenten der Phenylgruppe unabhängig voneinander Halogen, Trihalogenomethyl, Alkoxy, Alkyl oder Cyano sind; Heteroaryl für Indolyl, in Stellung 1 durch Alkyl oder Benzyl substituiertes Indolyl, Pyridyl, Pyrrolyl, Imidazolyl oder Thienyl steht; und die Alkyl- bzw. Alkoxygruppe 1 bis 3 Kohlenstoffatome enthält) bedeutet;

$A^1$ D- oder L-Alanin (Ala), (D- oder L-Valin (Val), D- oder L-Leucin (Leu), D- oder L-isoLeucin (Ile), D- oder L-Serin (Ser), D-oder L-Threonin (Thr), D- oder L-alloThreonin, D- oder L-Cystein (Cys), D-oder L-Methionin (Met), D- oder L-Phenylalanin (Phe), D- oder L-Tryptophan (Trp), N-Formyl geschütztes Trp, D- oder L-Tyrosin (Tyr), D- oder L- Prolin (Pro), D-oder L-Didehydroprolin (ΔPro) wie beispielsweise 3,4-Didehydroprolin (Δ(3,4)-Pro), D- oder L-Hydroxyprolin (Pro(OH)) wie beispielsweise 3-Hydroxyprolin (Pro(30H)) und 4-Hydroxyprolin (Pro(40H)), D- oder L-Azetidin-2-carbonsäure (Azt), D- oder L-Thioprolin (Tpr), D- oder L- Aminoprolin (Pro $(NH_2)$) wie beispielsweise 3-Aminoprolin (Pro($3NH_2$)) und 4-Aminoprolin (Pro($4NH_2$)), D- oder L- Pyroglutaminsäure (pGlu), D- oder L-2-Aminoisobuttersäure (Aib), D- oder L-2,3-Diaminopropionsäure, D- oder L-2,4-Diaminobuttersäure, D- oder L-Glutaminsäure (Glu), D- oder L-Asparaginsäure (Asp), D- oder L-Glutamin (Gln), D- oder L-Asparagin (Asn), D- oder L-Lysin (Lys), D- oder L-Arginin (Arg), D- oder L-Histidin (His), D- oder L-Ornithin (Orn), D- oder L-Hydroxypiperidincarbonsäure wie beispielsweise 5-Hydroxypiperidin-2-carbonsäure, D- oder L-Mercaptoprolin (Pro(SH)) wie beispielsweise 3-Mercaptoprolin (Pro(3SH)) und 4-Mercaptoprolin (Pro(4SH)), Tpr(O), Met(O), Tpr($O_2$) oder Met($O_2$), bedeutet und deren geometrische Isomere, wobei enthaltene Hydroxy- und Aminogruppen durch übliche Schutzgruppen (z.B. Acyl, Carbamoyl oder Aralkyl (insbesondere Benzyl) geschützt sein können.

**3.** Verbindung nach Anspruch 1 oder 2, worin

$R^1$ Aryl, Heteroaryl, Aralkyl, Heteroaralkyl, Aryloxyalkyl, Arylalkyloxy, Di(arylalkyl)aminoalkyl (worin Aryl für Phenyl oder 1- oder 2-fach substituiertes Phenyl steht; die Substituenten der Phenylgruppe unabhängig voneinander Halogen oder Alkoxy sind; Heteroaryl für Indolyl, in Stellung 1 durch Alkyl oder Benzyl substituiertes Indolyl oder Pyridyl steht; und die Alkyl- bzw. Alkoxygruppe 1 bis 3 Kohlenstoffatome enthält) bedeutet, insbesondere

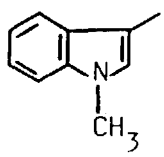

oder

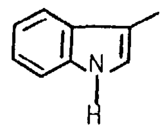

,

vorzugsweise

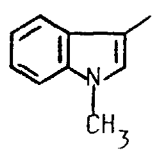

.

**4.** Verbindung nach Anspruch 1, 2 oder 3, worin $A^1$ eine Aminosäure ist, die eine oder 2 polare funktionelle Gruppe (n) in der Seitenkette trägt, wie OH, COOH, $NH_2$, Guanidin, $CONH_2$, SH.

**5.** Verbindung nach Anspruch 4, worin die funktionelle Gruppe in der Seitenkette von $A^1$ OH ist.

**6.** Verbindung nach Anspruch 1, 2 oder 3, worin $A^1$ Ser, Thr, Trp(For) oder Tyr ist.

**7.** Verbindung nach Anspruch 1, 2 oder 3, worin $A^1$ Pro oder 4-Hydroxyprolin ist.

**8.** Verbindung nach Anspruch 7, worin $A^1$ 4-Hydroxyprolin mit 2-S-Konfiguration ist, insbesondere

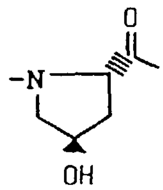

9. Verbindung nach einem der Ansprüche 1 bis 8, worin $R^5$ eine Gruppe der allgemeinen Formel II

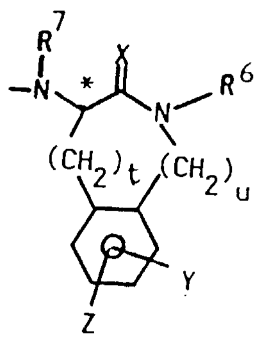

II

ist.

10. Verbindung nach Anspruch 9, worin t eins und u null oder t zwei und u null oder t und u je eins ist, und $R^6$, $R^7$, X, Y und Z wie in Anspruch 1 definiert sind.

11. Verbindung nach einem der Ansprüche 1 bis 10, worin $R^6$ Benzyl oder Methoxybenzyl ist.

12. Verbindung nach einem der Ansprüche 1 bis 11, worin $R^7$ Wasserstoff ist.

13. Verbindung nach einem der Ansprüche 1 bis 12, worin X Oxo ist.

14. Verbindung nach einem der Ansprüche 1 bis 13, worin Y und Z unabhängig voneinander Methoxy, Wasserstoff, $CF_3$ oder tert. Butyl sind oder zusammen $-(CH)_4-$ bedeuten.

15. Verfahren zur Herstellung einer Verbindung nach einem der Ansprüche 1 bis 14 oder deren Salze, dadurch gekennzeichnet, daß man nach bekannten Methoden schrittweise die jeweiligen Aminosäuren, beziehungsweise Peptidderivatteilsequenzen, Carbonsäuren und Amine kondensiert und die so erhaltene Verbindung in freier Form oder in Form des gewünschten Salzes isoliert.

16. Pharmazeutische Zubereitung enthaltend eine Verbindung nach einem der Ansprüche 1 bis 14.

17. Verwendung einer Verbindung nach einem der Ansprüche 1 bis 14 für die Herstellung einer pharmazeutischen Zubereitung zur Therapie von und zur Vorbeugung gegenüber Neurokinin-vermittelten Krankheiten.

**Claims**

1. Amino acid derivative of general formula I

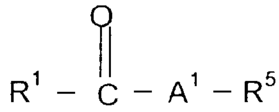 (I)

or a pharmaceutically acceptable salt thereof, wherein

$R^1$ is vinyl, aryl, heteroaryl, aralkyl, heteroaralkyl, arylvinyl, heteroarylvinyl, aryloxyalkyl, arylalkyloxy, $(C_{3\text{-}8})$ cycloalkyl, $(C_{3\text{-}8})$cycloalkylalkyl, bicycloheptyl or bicycloheptylalkyl (either unsubstituted or substituted by 1-3 methyl groups), adamantyl, adamantylalkyl, decaline, decalinalkyl, tetraline, tetralinalkyl, diphenylalkyl, di(arylalkyl)aminoalkyl or arylalkylaminoalkyl (wherein aryl is phenyl, mono-, di- or trisubstituted phenyl or naphthyl; the substituents of the phenyl group, independently of each other, represent halogen, trihalomethyl, alkoxy, alkyl, hydroxy, nitro, alkylcarbonyl or cyano; heteroaryl is indolyl, indolyl substituted in position 1 by alkyl or benzyl, pyridyl, pyrrolyl, imidazolyl or thienyl; and the alkyl or alkoxy group contains 1 to 3 carbon atoms;

$A^1$ is D- or L-alanine (Ala), (D- or L-valine (Val), D- or L-leucine (Leu), D- or L-isoleucine (Ile), D- or L-serine (Ser), D- or L-threonine (Thr), D- or L-allothreonine, D- or L-cysteine (Cys), D- or L-methionine (Met), D- or L-phenylalanine (Phe), D-or L-tryptophan (Trp), N-formyl protected Trp, D- or L-tyrosine (Tyr), D- or L-proline (Pro), D- or L-didehydroproline (ΔPro) such as 3,4-didehydroproline (Δ(3,4)-Pro), D- or L-hydroxyproline (Pro (OH)) such as 3-hydroxyproline (Pro(30H)) and 4-hydroxyproline (Pro(40H)), D- or L-azetidine-2-carboxylic acid (Azt), D- or L-thioproline (Tpr), D- or L-aminoproline [Pro($NH_2$)) such as 3-aminoproline (Pro(3$NH_2$)) and 4-aminoproline (Pro(4$NH_3$)), D- or L-pyroglutamic acid (pGlu), D- or L-2-aminoisobutyric acid (Aib), D- or L-2,3-diaminopropionic acid, D- or L-2,4-diaminobutyric acid, D- or L-glutamic acid (Glu), D- or L-aspartic acid (Asp), D- or L-glutamine (Gln), D- or L-asparagine (Asn), D- or L-lysine (Lys), D- or L-arginine (Arg), D- or L-histidine (His), D- or L-ornithine (Orn), D- or L-hydroxy piperidine carboxylic acid such as 5-hydroxypiperidine-2-carboxylic acid, D- or L-mercaptoproline (Pro(SH)) such as 3-mercaptoproline (Pro(3SH)) and 4-mercaptoproline (Pro(4SH)), Tpr(O), Met(O), Tpr($O_2$) or Met($O_2$), and the geometric isomers thereof, whereby the hydroxy and amino groups contained therein may be protected by conventional protecting groups (e.g. acyl, carbamoyl or aralkyl (in particular benzyl));

$R^5$ is an amine of formula

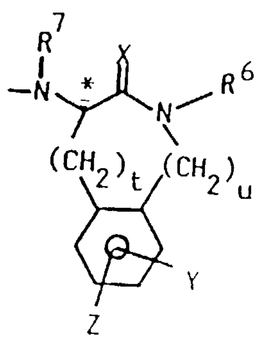

or

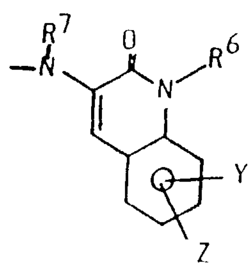

II

III

wherein

$R^6$ is aralkyl, diarylalkyl (in these groups aryl is phenyl or naphthyl and alkyl $(C_{1\text{-}5})$alkyl), heteroaryl-$(C_{1\text{-}5})$alkyl (wherein heteroaryl is 2-, 3- or 4-pyridyl or 2- or 3-thienyl), phenylamino-$(C_{1\text{-}5})$alkyl, nephthylamino- $(C_{1\text{-}5})$ alkyl or

N-phenylalkylpiperidinyl (wherein the phenyl groups listed are not substituted or contain 1, 2 or 3 substituents which, independently of each other, denote $(C_{1\text{-}5})$alkyl, preferably methyl, $(C_{1\text{-}5})$alkoxy, preferably methoxy, dimethylamine, halogen, trifluoromethyl, -CN or $OCF_3$) ;

$R_7$ is hydrogen or $(C_{1-5})$-alkyl;

X is 0 or $H_2$ ;

Y and Z independently of each other are hydrogen, $(C_{1-5})$alkyl, $(C_{1-5})$alkyloxy, benzyloxy (wherein the phenyl group is not substituted or contains 1, 2 or 3 substituents which, independently of each other, denote $(C_{1-5})$ alkyl, preferably methyl, $(C_{1-5})$alkoxy, preferably methoxy, dimethylamine, halogen, trifluoromethyl, -CN or $OCF_3$), $OCF_3$, halogen, $CF_3$, CN, $CH_2NH_2$, $CONH_2$, N-$(C_{1-5}$-alkyl$)_2$, NH-$(C_{1-4})$alkylcarbonyl, N-$(C_{1-5})$alkyl-N-$(C_{1-4})$alkylcarbonyl, $NH_2$ or NH-$(C_{1-5})$alkyl or if Y and Z are in a vicinal position to one another, both together represent $-OCH_2O-$, $OCH_2CH_2O-$ or $(CH)_4$;

t and u have one of the following meanings

(a) t and u are zero
(b) t is one and u is zero
(c) t and u are each one
(d) t is two and u is zero;

and if t is one and u is zero, $R^5$ is also an amine of formula IV

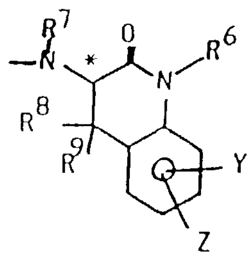

IV

wherein

$R^6$, $R^7$, Y and Z have the above meanings and

$R^8$ is hydrogen and $R^9$ is hydroxy, $(C_{1-5})$alkoxy, phenyl-$(C_{1-5})$alkyloxy, naphthyl-$(C_{1-5})$alkyloxy or $(C_{1-4})$alkylcarbonyl, or wherein

$R^8$ and $R^9$ are both oxygen or $-OCH_2CH_2O-$;

and the chirality to C* may be R or S.

**2.** Compound according to claim 1, wherein

$R^1$ is vinyl, aryl, heteroaryl, aralkyl, heteroaralkyl, arylvinyl, heteroarylvinyl, aryloxyalkyl, arylalkyloxy, di(arylalkyl) aminoalkyl or arylalkylaminoalkyl (wherein aryl is phenyl, mono-, di- or trisubstituted phenyl or naphthyl; the substituents of the phenyl group, independently of each other, are halogen, trihalomethyl, alkoxy, alkyl or cyano; heteroaryl is indolyl, indolyl substituted in position 1 by alkyl or benzyl, pyridyl, pyrrolyl, imidazolyl or thienyl; and the alkyl or alkoxy group contains 1 to 3 carbon atoms);

$A^1$ is D- or L-alanine (Ala), D- or L-valine (Val), D- or L-leucine (Leu), D- or L-isoleucine (Ile), D- or L-serine (Ser), D- or L-threonine (Thr), D- or L-allothreonine, D- or L-cysteine (Cys), D- or L-methionine (Met), D- or L-phenylalanine (Phe), D- or L-tryptophan (Trp), N-formyl protected Trp, D- or L-tyrosine (Tyr), D- or L-proline (Pro), D- or L-didehydroproline (ΔPro) such as 3,4-didehydroproline (Δ(3,4)-Pro), D- or L-hydroxyproline (Pro(OH)) such as 3-hydroxyproline (Pro(30H)) and 4-hydroxyproline (Pro(40H)), D- or L-azetidine-2-carboxylic acid (Azt), D- or L-thioproline (Tpr), D- or L-aminoproline (Pro(NH)) such as 3-aminoproline (Pro($3NH_2$)) and 4-aminoproline (Pro($4NH_2$)), D- or L-pyroglutamic acid (pGlu), D- or L-2-aminoisobutyric acid (Aib), D- or L-2,3-diaminopropionic acid, D- or L-2,4-diaminobutyric acid, D- or L-glutamic acid (Glu), D- or L-aspartic acid (Asp),

D- or L-glutamine (Gln), D- or L-asparagine (Asn), D- or L-lysine (Lys), D- or L-arginine (Arg), D- or L-histidine (His), D- or L-ornithine (Orn), D- or L-hydroxy piperidine carboxylic acid such as 5-hydroxypiperidine-2-carboxylic acid, D- or L-mercaptoproline (Pro(SH)) such as 3-mercaptoproline (Pro(3SH)) and 4-mercaptoproline (Pro(4SH)), Tpr(O), Met(O), Tpr($O_2$) or Met($O_2$), and the geometric isomers thereof, whereby the hydroxy and amino groups contained therein may be protected by conventionl protecting groups (e.g. acyl, carbamoyl or aralkyl (in particular benzyl)).

**3.** A compound according to claim 1 or 2 wherein

$R^1$ represents aryl, heteroaryl, aralkyl, heteroaralkyl, aryloxyalkyl, arylalkyloxy, di(arylalkyl)aminoalkyl (wherein aryl denotes phenyl or mono- or disubstituted phenyl; the substituents of the phenyl group independently of each other are halogen or alkoxy; heteroaryl denotes indolyl, indolyl substituted by alkyl or benzyl in position 1; and the alkyl or alkoxy group contains 1 to 3 carbon atoms), particularly

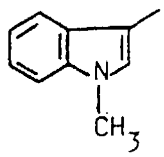

or

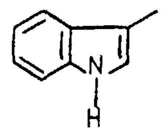

,

preferably

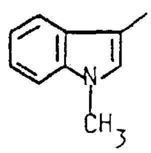

.

**4.** A compound according to claim 1, 2 or 3, wherein $A^1$ is an amino acid which carries one or 2 polar functional group (s) in the side chain such as OH, COOH, $NH_2$, guanidine, $CONH_2$, SH.

**5.** A compound according to claim 4, wherein the functional group in the side chain of $A^1$ is OH.

**6.** A compound according to claim 1, 2 or 3, wherein $A^1$ is Ser, Thr, Trp(For) or Tyr.

**7.** A compound according to claim 1, 2 or 3, wherein $A^1$ is Pro or 4-hydroxyproline.

**8.** A compound according to claim 7, wherein $A^1$ is 4-hydroxyproline with 2-S-configuration, more particularly

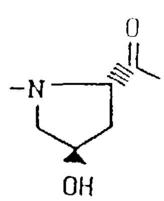

**9.** A compound according to any one of claims 1 to 8, wherein $R^5$ is a group of general formula II

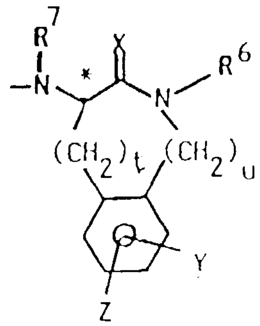

II

10. A compound according to claim 9, wherein t is one and u is zero or t is two and u is zero or t and u are each one, and $R^6$, $R^7$, X, Y and Z are defined as in claim 1.

11. A compound according to any one of claims 1 to 10, wherein $R^6$ is benzyl or methoxybenzyl.

12. A compound according to any one of claims 1 to 11, wherein $R^7$ is hydrogen.

13. A compound according to any one of claims 1 to 12, wherein X is Oxo.

14. A compound according to any one of claims 1 to 13, wherein Y and Z independently of each other are methoxy, hydrogen, $CF_3$ or tert.butyl or together denote $-(CH)_4-$.

15. A process for preparing a compound according to any one of claims 1 to 14 or the salts thereof, characterised in that the relevant amino acids, or part sequences of peptide derivatives, carboxylic acids and amines are concentrated step by step, using known methods, and the resultant compound is isolated in free form or in the form of the desired salt.

16. A pharmaceutical preparation containing a compound according to one of claims 1 to 14.

17. Use of a compound according to one of claims 1 to 14 for producing a pharmaceutical prepparation for treating and preventing neurokinin-mediated illnesses.

**Revendications**

1. Dérivé d'acide aminé de formule générale I

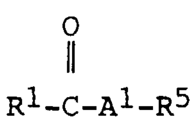

(I)

ou son sel pharmaceutiquement acceptable, où

$R^1$ représente vinyle, aryle, hétéroaryle, aralkyle, hétéroaralkyle, arylvinyle, hétéroarylvinyle, aryloxyalkyle, arylalkyloxy, cycloalkyle ($C_3$-$C_8$), cycloalkyl-($C_3$-$C_8$)alkyle, bicycloheptyle ou bicycloheptylalkyle non substitué ou substitué par 1-3 groupes méthyle, adamantyle, adamantylalkyle, décaline, décalinalkyle, tétraline, tétralinalkyle, diphénylalkyle, di(arylalkyl)aminoalkyle ou arylalkylaminoalkyle (où aryle représente phényle, phényle 1-, 2- ou 3-fois substitué ou naphtyle; les substituants du groupe phényle étant indépendamment les uns des autres halogène, trihalogénométhyle, alcoxy, alkyle, hydroxyle, nitro, alkylcarbonyle ou cyano; hétéroaryle représente indolyle, indolyle substitué en position 1 par alkyle ou benzyle, pyridyle, pyrrolyle, imidazolyle ou thiényle; et le groupe alkyle ou alcoxy contient 1 à 3 atomes de carbone);

$A^1$ représente D- ou L-alanine (Ala), (D- ou L-valine (Val), D- ou L-leucine (Leu), D- ou L-isoleucine (Ile), D- ou L-sérine (Ser), D- ou L-thréonine (Thr), D- ou L-allothréonine, D- ou L-cystéine (Cys), D- ou L-méthionine (Met), D- ou L-phénylalanine (Phe), D- ou L-tryptophane (Trp), Trp protégé par N-formyle, D- ou L-tyrosine (Tyr), D- ou L-proline (Pro), D- ou L-didéshydroproline (ΔPro) comme par exemple 3,4-didéshydroproline (Δ(3,4)-Pro), D- ou L-hydroxyproline (Pro(OH)) comme par exemple 3-hydroxyproline (Pro(30H)) et 4-hydroxyproline (Pro(40H)), acide D- ou L-azétidine-2-carboxylique (Azt), D- ou L-thioproline (Tpr), D- ou L-aminoproline ($Pro(NH_2)$) comme par exemple 3-aminoproline ($Pro(3NH_2)$) et 4-aminoproline ($Pro(4NH_2)$), acide D- ou L-pyroglutamique (pGlu), acide D- ou L-2-aminoisobutyrique (Aib), acide D- ou L-2,3-diaminopropionique, acide D- ou L-2,4- diaminobutyrique, acide D- ou L-glutamique (Glu), acide D- ou L- aspartique (Asp), D- ou L-glutamine (Gln), D- ou L-asparagine (Asn), D- ou L-lysine (Lys), D- ou L-arginine (Arg), D- ou L-histidine (His), D- ou L-ornithine (Orn), acide D- ou L-hydroxypipéridinecarboxylique comme par exemple acide 5-hydroxy-pipéridine-2-carboxylique, D- ou L-mercaptoproline (Pro(SH)) comme par exemple 3-mercaptoproline (Pro(3SH)) et 4-mercaptoproline (Pro(4SH)), Tpr(O), Met(O), $Tpr(O_2)$ ou $Met(O_2)$, et leurs isomères géométriques, où les groupes hydroxyle et amino contenus peuvent être protégés par des groupes protecteurs courants (par exemple acyle, carbamoyle ou aralkyle (en particulier benzyle);

$R^5$ représente une amine de formule

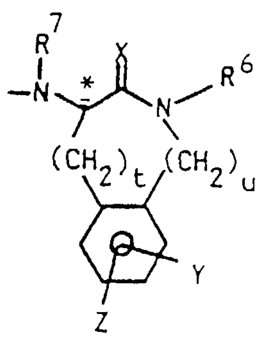

II

ou

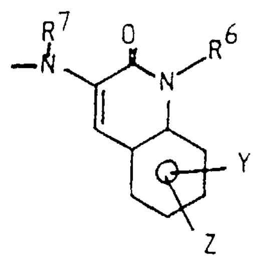

III

où

$R^6$ représente aralkyle, diarylalkyle (dans ces groupes aryle est phényle ou naphtyle et alkyle est alkyle($C_1$-$C_5$)), hétéroaryl-alkyle($C_1$-$C_5$) (où hétéroaryle est 2-, 3- ou 4-pyridyle ou 2- ou 3-thiényle), phénylaminoalkyle($C_1$-$C_5$), naphtylamino-alkyle($C_1$-$C_5$) ou N-phénylalkylpipéridinyle (où les groupes phényle indiqués sont non substitués ou contiennent 1, 2 ou 3 substituants qui sont indépendamment les uns des autres alkyle($C_1$-$C_5$), de préférence méthyle, alcoxy($C_1$-$C_5$), de préférence méthoxy, diméthylamine, halogène, trifluorométhyle, -CN ou $OCF_3$);

$R^7$ représente l'hydrogène ou alkyle($C_1$-$C_5$);

X représente O ou $H_2$;

Y et Z représentent indépendamment l'un de l'autre hydrogène, alkyle($C_1$-$C_5$), alkyloxy($C_1$-$C_5$), benzyloxy (où le groupe phényle est non substitué ou contient 1, 2 ou 3 substituants qui sont indépendamment les uns des autres alkyle($C_1$-$C_5$), de préférence méthyle, alcoxy($C_1$-$C_5$), de préférence méthoxy, diméthylamine, halogène, trifluorométhyle, -CN ou $OCF_3$), $OCF_3$, halogène, $CF_3$, CN, $CH_2NH_2$, $CONH_2$, N-(alkyle($C_1$-$C_5$))$_2$, NH-alkyl ($C_1$-$C_4$)carbonyle, N-alkyl($C_1$-$C_5$)-N-alkyl($C_1$-$C_4$)carbonyle, $NH_2$ ou NH-alkyle($C_1$-$C_5$) ou quand Y et Z sont voisins l'un de l'autre, ceux-ci représentent ensemble $-OCH_2O-$, $-OCH_2CH_2O-$ ou (CH)4;

t et u ont l'une des significations suivantes:

(a) t et u sont zéro
(b) t est un et u est zéro
(c) t et u sont chacun un
(d) t est deux et u est zéro;

et quand t est un et u est zéro, $R^5$ représente aussi une amine de formule IV

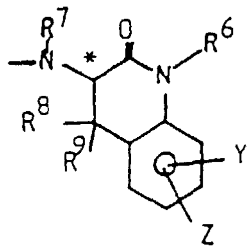

IV

où

$R^6$, $R^7$, Y et Z ont la signification indiquée ci-dessus et $R^8$ représente l'hydrogène et $R^9$ représente hydroxyle, alcoxy($C_1$-$C_5$), phényl-alkyloxy($C_1$-$C_5$), naphtyl-alkyloxy($C_1$-$C_5$) ou alkyl($C_1$-$C_4$)carbonyle, ou bien où $R^8$ et $R^9$ représentent ensemble l'oxygène ou $-OCH_2CH_2O-$; et la chiralité sur C* peut représenter R ou S.

**2.** Composé selon la revendication 1 où

$R^1$ représente vinyle, aryle, hétéroaryle, aralkyle, hétéroaralkyle, arylvinyle, hétéroarylvinyle, aryloxyalkyle, arylalkyloxy, di(arylalkyl)aminoalkyle ou arylalkylaminoalkyle (où aryle représente phényle, phényle L-, 2- ou 3-fois substitué ou naphtyle; les substituants du groupe phényle étant indépendamment les uns des autres halogène, trihalogénométhyle, alcoxy, alkyle ou cyano; hétéroaryle représente indolyle, indolyle substitué en position 1 par alkyle ou benzyle, pyridyle, pyrrolyle, imidazolyle ou thiényle; et le groupe alkyle ou alcoxy contient 1 à 3 atomes de carbone);

$A^1$ représente D- ou L-alanine (Ala), D- ou L-valine (Val), D- ou L-leucine (Leu), D- ou L-isoleucine (Ile), D- ou L-sérine (Ser), D- ou L-thréonine (Thr), D- ou L-allothréonine, D- ou L-cystéine (Cys), D- ou L-méthionine (Met), D- ou L-phénylalanine (Phe), D- ou L-tryptophane (Trp), Trp protégé par N-formyle, D- ou L-tyrosine (Tyr), D- ou L-proline (Pro), D- ou L-didéshydroproline (ΔPro) comme par exemple 3,4-didéshydroproline (Δ (3,4)-Pro), D- ou L- hydroxyproline (Pro(OH)) comme par exemple 3-hydroxyproline (Pro(3OH)) et 4-hydroxyproline (Pro(4OH)), acide D- ou L-azétidine-2-carboxylique (Azt), D- ou L-thioproline (Tpr), D- ou L-aminoproline (Pro($NH_2$)) comme par exemple 3-aminoproline (Pro($3NH_2$)) et 4-aminoproline (Pro($4NH_2$)), acide D- ou L-pyroglutamique (pGlu), acide D- ou L-2-aminoisobutyrique (Aib), acide D- ou L-2,3-diaminopropionique, acide D- ou L-2,4-diaminobutyrique, acide D- ou L-glutamique (Glu), acide D- ou L- aspartique (Asp), D- ou L-glutamine (Gln), D-ou L- asparagine (Asn), D- ou L-lysine (Lys), D- ou L-arginine (Arg), D- ou L-histidine (His), D- ou L-ornithine (Orn), acide D- ou L-hydroxypipéridinecarboxylique comme par exemple acide 5-hydroxypipéridine-2-carboxylique, D- ou L-mercaptoproline (Pro(SH)) comme par exemple 3-mercaptoproline (Pro(3SH)) et 4-mercaptoproline (Pro(4sH)), Tpr(O), Met(O), Tpr($O_2$) ou Met($O_2$), et leurs isomères géométriques, où les groupes hydroxyle et amino contenus peuvent être protégés par des groupes protecteurs courants (par exemple acyle, carbamoyle ou aralkyle (en particulier benzyle).

**3.** Composé selon la revendication 1 ou 2 où

$R^1$ représente aryle, hétéroaryle, aralkyle, hétéroaralkyle, aryloxyalkyle, arylalkyloxy, di(arylalkyl)aminoalkyle (où aryle représente phényle ou phényle 1 ou 2 fois substitué; les substituants du groupe phényle sont indépendamment l'un de l'autre halogène ou alcoxy; hétéroaryle représente indolyle, indolyle substitué en position 1 par alkyle ou benzyle, ou pyridyle; et le groupe alkyle ou alcoxy contient 1 à 3 atomes de carbone), en particulier

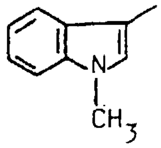

ou

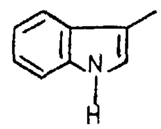

,

de préférence

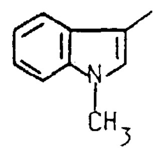

.

4. Composé selon la revendication 1, 2 ou 3 où $A^1$ est un acide aminé qui porte un ou deux groupes fonctionnels polaires dans la chaîne latérale, comme OH, COOH, $NH_2$, guanidine, $CONH_2$, SH.

5. Composé selon la revendication 4 où le groupe fonctionnel dans la chaîne latérale de $A^1$ est OH.

6. Composé selon la revendication 1, 2 ou 3 où $A^1$ est Ser, Thr, Trp(For) ou Tyr.

7. Composé selon la revendication 1, 2 ou 3 où $A^1$ est Pro ou 4-hydroxyproline.

8. Composé selon la revendication 7 où $A^1$ est 4-hydroxyproline dans la configuration 2-S, en particulier

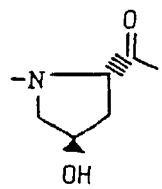

9. Composé selon l'une des revendications 1 à 8 où $R^5$ est un groupe de formule générale II

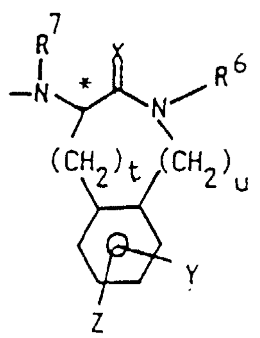

II

10. Composé selon la revendication 9 où t est un et u est zéro ou t est deux et u est zéro ou t et u sont chacun un, et $R^6$, $R^7$, X, Y et Z sont définis comme dans la revendication 1.

11. Composé selon l'une des revendications 1 à 10 où $R^6$ est benzyle ou méthoxybenzyle.

12. Composé selon l'une des revendications 1 à 11 où $R^7$ est hydrogène.

13. Composé selon l'une des revendications 1 à 12 où X est oxo.

14. Composé selon l'une des revendications 1 à 13 où Y et Z sont indépendamment l'un de l'autre méthoxy, hydrogène, $CF_3$ ou tert.butyle ou représentent ensemble $-(CH)_4-$.

15. Procédé de préparation d'un composé selon l'une des revendications 1 à 14 ou de ses sels, caractérisé en ce que l'on condense selon des procédés connus, par étapes, les différents acides aminés, ou les différentes séquences partielles de dérivés peptidiques, les différents acides carboxyliques et les différentes amines et on isole le composé ainsi obtenu sous forme libre ou sous forme du sel voulu.

**16.** Préparation pharmaceutique contenant un composé selon l'une des revendications 1 à 14.

**17.** Utilisation d'un composé selon l'une des revendications 1 à 14 pour la production d'une préparation pharmaceutique pour la thérapie et la prévention de maladies à médiation par des neurokinines.